# EUROPEAN PATENT APPLICATION

(11) **EP 3 229 026 A1**
(43) Date of publication of application: **11.10.2017**
(21) Application number: 16305403.4
(22) Date of filing: 06.04.2016
(51) Int. Cl.: G01N 33/68, G01N 33/50

(54) **BIOMARKERS FOR THE DIAGNOSIS OF INFLAMMATION-RELATED DISEASES**

(71) Applicant: UNIVERSITE DE BORDEAUX, 33000 Bordeaux (FR); Centre National de la Recherche Scientifique, 75794 Paris Cedex 16 (FR)
(72) Inventor: FAUSTIN, Benjamin, 33600 Pessac (FR)
(74) Representative: Grosset-Fournier, Chantal Catherine

(57) **Abstract**

The invention relates to new biomarkers for the diagnosis of inflammation-related diseases.

SEQUENCE LISTING

<110>  UNIVERSITE DE BORDEAUX
       CENTRE NATIONAL DE LA RECHERCHE SCIENTIFIQUE

<120>  BIOMARKERS FOR THE DIAGNOSIS OF INFLAMMATION-RELATED DISEASES

<130>  IOB 16 AA BRD FLAM

<160>  2

<170>  PatentIn version 3.5

<210>  1
<211>  3581
<212>  DNA
<213>  Homo sapiens

<400>  1
agaatgtcat cctcaaggga agtgcagaga gatttcttca gtcctcagct gagtataagc      60

tggcctcctg gagtctgtga acacaaacgt ccaatgtgag tgtgcctgtg caagcccctg     120

gctgtttata ctccggaggg tgtccccgtg cgtcatcggt ggagtggacc aaaactggtg     180

atctgtttgc cctgtgtgac cttgcccaga accctgctga ctgagagaac acatctgctg     240

gaagtcctct gggattcaag gtacaggaag aactcgaggc ctcactgaaa cggaaagcaa     300

atacaaagaa actttatttt aaaaacgtgt cttggtctcc caagaagagg gcaattggat     360

tgctcagcca gaatgaagag tagttttaca gaaaaaagag gacaatattg ggatcacctt     420

tgacctttcc atttggaaat aatattttct attgtgttat agaaaggtgg gaagctttca     480

tccagaacaa tgaatttcat aaaggacaat agccgagccc ttattcaaag aatgggaatg     540

actgttataa agcaaatcac agatgaccta tttgtatgga atgttctgaa tcgcgaagaa     600

gtaaacatca tttgctgcga gaaggtggag caggatgctg ctagagggat cattcacatg     660

attttgaaaa agggttcaga gtcctgtaac ctctttctta aatcccttaa ggagtggaac     720

tatcctctat ttcaggactt gaatggacaa agtctttttc atcagacatc agaaggagac     780

ttggacgatt tggctcagga tttaaaggac ttgtaccata ccccatcttt tctgaacttt     840

tatccccttg gtgaagatat tgacattatt tttaacttga aaagcacctt cacagaacca     900

gtcctgtgga ggaaggacca acaccatcac cgcgtggagc agctgaccct gaatggcctc     960

ctgcaggctc ttcagagccc ctgcatcatt gaaggggaat ctggcaaagg caagtccact    1020

ctgctgcagc gcattgccat gctctggggc tccggaaagt gcaaggctct gaccaagttc    1080

aaattcgtct tcttcctccg tctcagcagg gcccagggtg gactttttga aaccctctgt    1140

gatcaactcc tggatatacc tggcacaatc aggaagcaga cattcatggc catgctgctg    1200

aagctgcggc agagggttct tttccttctt gatggctaca atgaattcaa gccccagaac    1260

tgcccagaaa tcgaagccct gataaaggaa aaccaccgct tcaagaacat ggtcatcgtc    1320

accactacca ctgagtgcct gaggcacata cggcagtttg gtgccctgac tgctgaggtg 1380

ggggatatga cagaagacag cgcccaggct ctcatccgag aagtgctgat caaggagctt 1440

gctgaaggct tgttgctcca aattcagaaa tccaggtgct tgaggaatct catgaagacc 1500

cctctctttg tggtcatcac ttgtgcaatc cagatgggtg aaagtgagtt ccactctcac 1560

acacaaacaa cgctgttcca taccttctat gatctgttga tacagaaaaa caaacacaaa 1620

cataaaggtg tggctgcaag tgacttcatt cggagcctgg accactgtgg agacctagct 1680

ctggagggtg tgttctccca caagtttgat ttcgaactgc aggatgtgtc cagcgtgaat 1740

gaggatgccc tgctgacaac tgggctcctc tgtaaatata cagctcaaag gttcaagcca 1800

aagtataaat tctttcacaa gtcattccag gagtacacag caggacgaag actcagcagt 1860

ttattgacgt ctcatgagcc agaggaggtg accaagggga atggttactt gcagaaaatg 1920

gtttccattt cggacattac atccacttat agcagcctgc tccggtacac ctgtgggtca 1980

tctgtggaag ccaccagggc tgttatgaag cacctcgcag cagtgtatca acacggctgc 2040

cttctcggac tttccatcgc caagaggcct ctctggagac aggaatcttt gcaaagtgtg 2100

aaaaacacca ctgagcaaga aattctgaaa gccataaaca tcaattcctt tgtagagtgt 2160

ggcatccatt tatatcaaga gagtacatcc aaatcagccc tgagccaaga atttgaagct 2220

ttctttcaag gtaaaagctt atatatcaac tcagggaaca tccccgatta cttatttgac 2280

ttctttgaac atttgcccaa ttgtgcaagt gccctggact tcattaaact ggacttttat 2340

gggggagcta tggcttcatg ggaaaaggct gcagaagaca caggtggaat ccacatggaa 2400

gaggccccag aaacctacat tcccagcagg gctgtatctt tgttcttcaa ctggaagcag 2460

gaattcagga ctctggaggt cacactccgg gatttcagca agttgaataa gcaagatatc 2520

agatatctgg ggaaaatatt cagctctgcc acaagcctca ggctgcaaat aaagagatgt 2580

gctggtgtgg ctggaagcct cagtttggtc ctcagcacct gtaagaacat ttattctctc 2640

atggtggaag ccagtcccct caccatagaa gatgagaggc acatcacatc tgtaacaaac 2700

ctgaaaacct tgagtattca tgacctacag aatcaacggc tgccgggtgg tctgactgac 2760

agcttgggta acttgaagaa ccttacaaag ctcataatgg ataacataaa gatgaatgaa 2820

gaagatgcta taaaactagc tgaaggcctg aaaaacctga agaagatgtg tttatttcat 2880

ttgacccact tgtctgacat tggagaggga atggattaca tagtcaagtc tctgtcaagt 2940

gaaccctgtg accttgaaga aattcaatta gtctcctgct gcttgtctgc aaatgcagtg 3000

aaaatcctag ctcagaatct tcacaatttg gtcaaactga gcattcttga tttatcagaa 3060

aattacctgg aaaaagatgg aaatgaagct cttcatgaac tgatcgacag gatgaacgtg 3120

ctagaacagc tcaccgcact gatgctgccc tggggctgtg acgtgcaagg cagcctgagc 3180

agcctgttga aacatttgga ggaggtccca caactcgtca agcttgggtt gaaaaactgg 3240

agactcacag atacagagat tagaatttta ggtgcatttt ttggaaagaa ccctctgaaa 3300

aacttccagc agttgaattt ggcgggaaat cgtgtgagca gtgatggatg gcttgccttc 3360

atgggtgtat ttgagaatct taagcaatta gtgttttttg actttagtac taaagaattt 3420

ctacctgatc cagcattagt cagaaaactt agccaagtgt tatccaagtt aacttttctg 3480

caagaagcta ggcttgttgg gtggcaattt gatgatgatg atctcagtgt tattacaggt 3540

gcttttaaac tagtaactgc ttaaataaag tgtactcgaa g 3581

<210> 2
<211> 1024
<212> PRT
<213> Homo sapiens

<400> 2

Met Asn Phe Ile Lys Asp Asn Ser Arg Ala Leu Ile Gln Arg Met Gly
1               5                   10                  15

Met Thr Val Ile Lys Gln Ile Thr Asp Asp Leu Phe Val Trp Asn Val
            20                  25                  30

Leu Asn Arg Glu Glu Val Asn Ile Ile Cys Cys Glu Lys Val Glu Gln
        35                  40                  45

Asp Ala Ala Arg Gly Ile Ile His Met Ile Leu Lys Lys Gly Ser Glu
    50                  55                  60

Ser Cys Asn Leu Phe Leu Lys Ser Leu Lys Glu Trp Asn Tyr Pro Leu
65                  70                  75                  80

Phe Gln Asp Leu Asn Gly Gln Ser Leu Phe His Gln Thr Ser Glu Gly
                85                  90                  95

Asp Leu Asp Asp Leu Ala Gln Asp Leu Lys Asp Leu Tyr His Thr Pro
            100                 105                 110

Ser Phe Leu Asn Phe Tyr Pro Leu Gly Glu Asp Ile Asp Ile Ile Phe
        115                 120                 125

Asn Leu Lys Ser Thr Phe Thr Glu Pro Val Leu Trp Arg Lys Asp Gln
    130                 135                 140

His His His Arg Val Glu Gln Leu Thr Leu Asn Gly Leu Leu Gln Ala
145                 150                 155                 160

Leu Gln Ser Pro Cys Ile Ile Glu Gly Glu Ser Gly Lys Gly Lys Ser
                165                 170                 175

Thr Leu Leu Gln Arg Ile Ala Met Leu Trp Gly Ser Gly Lys Cys Lys
            180                 185                 190

Ala Leu Thr Lys Phe Lys Phe Val Phe Phe Leu Arg Leu Ser Arg Ala
        195                 200                 205

Gln Gly Gly Leu Phe Glu Thr Leu Cys Asp Gln Leu Leu Asp Ile Pro
    210                 215                 220

Gly Thr Ile Arg Lys Gln Thr Phe Met Ala Met Leu Leu Lys Leu Arg
225                 230                 235                 240

Gln Arg Val Leu Phe Leu Leu Asp Gly Tyr Asn Glu Phe Lys Pro Gln
                245                 250                 255

Asn Cys Pro Glu Ile Glu Ala Leu Ile Lys Glu Asn His Arg Phe Lys
            260                 265                 270

Asn Met Val Ile Val Thr Thr Thr Thr Glu Cys Leu Arg His Ile Arg
        275                 280                 285

Gln Phe Gly Ala Leu Thr Ala Glu Val Gly Asp Met Thr Glu Asp Ser
    290                 295                 300

Ala Gln Ala Leu Ile Arg Glu Val Leu Ile Lys Glu Leu Ala Glu Gly
305                 310                 315                 320

Leu Leu Leu Gln Ile Gln Lys Ser Arg Cys Leu Arg Asn Leu Met Lys
                325                 330                 335

Thr Pro Leu Phe Val Val Ile Thr Cys Ala Ile Gln Met Gly Glu Ser
            340                 345                 350

Glu Phe His Ser His Thr Gln Thr Thr Leu Phe His Thr Phe Tyr Asp
        355                 360                 365

Leu Leu Ile Gln Lys Asn Lys His Lys His Lys Gly Val Ala Ala Ser
    370                 375                 380

Asp Phe Ile Arg Ser Leu Asp His Cys Gly Asp Leu Ala Leu Glu Gly
385                 390                 395                 400

Val Phe Ser His Lys Phe Asp Phe Glu Leu Gln Asp Val Ser Ser Val
                405                 410                 415

Asn Glu Asp Ala Leu Leu Thr Thr Gly Leu Leu Cys Lys Tyr Thr Ala

            420                 425                 430

Gln Arg Phe Lys Pro Lys Tyr Lys Phe Phe His Lys Ser Phe Gln Glu
        435                 440                 445

Tyr Thr Ala Gly Arg Arg Leu Ser Ser Leu Leu Thr Ser His Glu Pro
    450                 455                 460

Glu Glu Val Thr Lys Gly Asn Gly Tyr Leu Gln Lys Met Val Ser Ile
465                 470                 475                 480

Ser Asp Ile Thr Ser Thr Tyr Ser Ser Leu Leu Arg Tyr Thr Cys Gly
                485                 490                 495

Ser Ser Val Glu Ala Thr Arg Ala Val Met Lys His Leu Ala Ala Val
            500                 505                 510

Tyr Gln His Gly Cys Leu Leu Gly Leu Ser Ile Ala Lys Arg Pro Leu
        515                 520                 525

Trp Arg Gln Glu Ser Leu Gln Ser Val Lys Asn Thr Thr Glu Gln Glu
    530                 535                 540

Ile Leu Lys Ala Ile Asn Ile Asn Ser Phe Val Glu Cys Gly Ile His
545                 550                 555                 560

Leu Tyr Gln Glu Ser Thr Ser Lys Ser Ala Leu Ser Gln Glu Phe Glu
                565                 570                 575

Ala Phe Phe Gln Gly Lys Ser Leu Tyr Ile Asn Ser Gly Asn Ile Pro
            580                 585                 590

Asp Tyr Leu Phe Asp Phe Phe Glu His Leu Pro Asn Cys Ala Ser Ala
        595                 600                 605

Leu Asp Phe Ile Lys Leu Asp Phe Tyr Gly Gly Ala Met Ala Ser Trp
    610                 615                 620

Glu Lys Ala Ala Glu Asp Thr Gly Gly Ile His Met Glu Glu Ala Pro
625                 630                 635                 640

Glu Thr Tyr Ile Pro Ser Arg Ala Val Ser Leu Phe Phe Asn Trp Lys
                645                 650                 655

Gln Glu Phe Arg Thr Leu Glu Val Thr Leu Arg Asp Phe Ser Lys Leu
            660                 665                 670

Asn Lys Gln Asp Ile Arg Tyr Leu Gly Lys Ile Phe Ser Ser Ala Thr
        675                 680                 685

Ser Leu Arg Leu Gln Ile Lys Arg Cys Ala Gly Val Ala Gly Ser Leu
    690                 695                 700

Ser Leu Val Leu Ser Thr Cys Lys Asn Ile Tyr Ser Leu Met Val Glu
705                 710                 715                 720

Ala Ser Pro Leu Thr Ile Glu Asp Glu Arg His Ile Thr Ser Val Thr
                725                 730                 735

Asn Leu Lys Thr Leu Ser Ile His Asp Leu Gln Asn Gln Arg Leu Pro
            740                 745                 750

Gly Gly Leu Thr Asp Ser Leu Gly Asn Leu Lys Asn Leu Thr Lys Leu
        755                 760                 765

Ile Met Asp Asn Ile Lys Met Asn Glu Glu Asp Ala Ile Lys Leu Ala
    770                 775                 780

Glu Gly Leu Lys Asn Leu Lys Lys Met Cys Leu Phe His Leu Thr His
785                 790                 795                 800

Leu Ser Asp Ile Gly Glu Gly Met Asp Tyr Ile Val Lys Ser Leu Ser
                805                 810                 815

Ser Glu Pro Cys Asp Leu Glu Glu Ile Gln Leu Val Ser Cys Cys Leu
            820                 825                 830

Ser Ala Asn Ala Val Lys Ile Leu Ala Gln Asn Leu His Asn Leu Val
        835                 840                 845

Lys Leu Ser Ile Leu Asp Leu Ser Glu Asn Tyr Leu Glu Lys Asp Gly
    850                 855                 860

Asn Glu Ala Leu His Glu Leu Ile Asp Arg Met Asn Val Leu Glu Gln
865                 870                 875                 880

Leu Thr Ala Leu Met Leu Pro Trp Gly Cys Asp Val Gln Gly Ser Leu
                885                 890                 895

Ser Ser Leu Leu Lys His Leu Glu Glu Val Pro Gln Leu Val Lys Leu
            900                 905                 910

Gly Leu Lys Asn Trp Arg Leu Thr Asp Thr Glu Ile Arg Ile Leu Gly
        915                 920                 925

Ala Phe Phe Gly Lys Asn Pro Leu Lys Asn Phe Gln Gln Leu Asn Leu
    930                 935                 940

Ala Gly Asn Arg Val Ser Ser Asp Gly Trp Leu Ala Phe Met Gly Val
945                 950                 955                 960

Phe Glu Asn Leu Lys Gln Leu Val Phe Phe Asp Phe Ser Thr Lys Glu
                965                 970                 975

Phe Leu Pro Asp Pro Ala Leu Val Arg Lys Leu Ser Gln Val Leu Ser
            980                 985                 990

Lys Leu Thr Phe Leu Gln Glu Ala  Arg Leu Val Gly Trp  Gln Phe Asp
        995                 1000                1005

Asp Asp  Asp Leu Ser Val Ile  Thr Gly Ala Phe Lys  Leu Val Thr
    1010                1015                1020

Ala

## Description

The invention relates to new biomarkers for the diagnosis of inflammation-related diseases.

Inflammation, in particular low-grade chronic inflammation, is characteristic of many diseases of aging, including cardiovascular conditions, but the mechanisms remain unclear (Howcroft et al., The role of inflammation in age-related disease. Aging 5, 84-93, 2013; Okin et al., Evolution of inflammatory diseases. Current biology: CB 22, R733-740, 2012; Scrivo et al., Inflammation as "common soil" of the multifactorial diseases. Autoimmunity reviews 10, 369-374, 2011).

The inflammasomes are important determinants of inflammation. These macromolecular structures composed of NOD-like receptors (NLRs) or the "Absent in Melanoma 2" (AIM2) protein recognize specific cytosolic determinants produced by pathogens or cellular stress and trigger the caspase-1-dependent maturation and the secretion of interleukin-1 family cytokines (IL1FC) (Martinon et al., The inflammasome: a molecular platform triggering activation of inflammatory caspases and processing of proIL-beta. Molecular cell 10, 417-426, 2002).

The IL1FC includes potent inflammatory cytokines, which are found at higher levels in some older people (Furman et al., Apoptosis and other immune biomarkers predict influenza vaccine responsiveness. Molecular systems biology 9, 659, 2013) and have been directly linked to an increased risk of cardiovascular disease (Duewell et al., NLRP3 inflammasomes are required for atherogenesis and activated by cholesterol crystals. Nature 464, 1357-1361, 2010), cancer (Zitvogel et al., Inflammasomes in carcinogenesis and anticancer immune responses. Nature immunology 13, 343-351, 2012), functional decline (Youm et al., Canonical NIrp3 inflammasome links systemic low-grade inflammation to functional decline in aging. Cell metabolism 18, 519-532, 2013) and other degenerative diseases (Sardi et al., Alzheimer's disease, autoimmunity and inflammation. The good, the bad and the ugly. Autoimmunity reviews 11, 149-153, 2011).

In a recent study of aging rats, the expression levels of multiple molecules associated with the activation of inflammasomes were significantly elevated, as were the levels of IL1FC (Song et al., The expression changes of inflammasomes in the aging rat kidneys. The journals of gerontology, Series A, Biological sciences and medical sciences, 2015). However, it is not known whether the inflammasomes are activated in human aging and whether such changes are clinically relevant.

Therefore, there is a need for biomarkers useful for identifying patients most at risk for inflammation-related diseases.

In this context, the purpose of the invention is to provide biomarkers useful for the diagnosis of inflammation-related diseases.

Another purpose of the invention is to provide a method for the diagnosis of inflammation-related diseases.

Another purpose of the invention is to provide a screening method for determining the efficacy of a compound for the treatment of inflammation-related diseases.

Another purpose of the invention is to provide a method of monitoring treatment efficacy in a subject undergoing treatment for inflammation-related diseases.

A further purpose is also to provide a kit for the diagnosis of inflammation-related diseases.

The present invention relates to the use of at least one nucleotide-derived metabolite as biomarkers useful for the diagnosis of inflammation-related diseases.

The present invention relates to the use of at least one nucleotide-derived metabolite selected from the group consisting of adenine and N4-acetylcytidine in the *in vitro*/*ex vivo* diagnosis of a disease in a sample obtained from a subject, said disease being an inflammation-related disease.

The invention is based on the unexpected observations made by the Inventors that the two circulating nucleotide-derived metabolites, adenine and N4-acetylcytidine, prime and activate the NLRC4 inflammasome (N4-acetylcytidine induces the expression of the NLRC4 protein and the adenine activates the NLRC4 inflammasome), and thus induce production of interleukines IL-1β and IL-18, activate platelets and elevate blood pressure *in vivo* in mice.

In the invention, the expression "refers to the two groups of nitrogenous bases derived from purine or pyrimidine respectively. These nitrogenous bases can be bound or not to a ribose (ribonucleoside) or a deoxyribose (deoxyribonucleoside).

In particular, the adenine is a purine derivative represented by the formula (i):

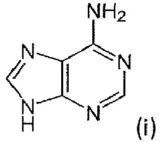

In particular, the N4-acetylcytidine is a pyrimidine derivative bound to a ribose represented by the formula (ii):

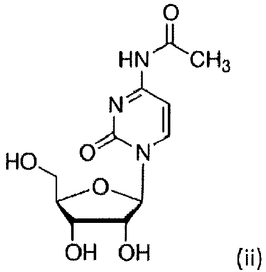

In the invention, the expression "inflammation-related disease" refers to diseases resulting directly or indirectly from the inflammatory response. Such diseases are not always considered to be inflammatory diseases but they are recognized as having an inflammatory component that contributes significantly to the disease process.

In an embodiment, the present invention relates to the use as defined above, wherein said disease is an inflammasome-related disease.

In the invention, the expression "inflammasome" refers to a protein complex build around several proteins, including NLRP3, NLRC4, AIM2 and NLRP6. In reply to a diverse range of microbial, stress and damage signals, this protein complex activates the caspase-1, which subsequently induces secretion of potent pro-inflammatory cytokines and cell death.

In the invention, the expression "inflammasome-related disease" refers to a group of inflammation-related diseases in which the inflammasome plays a critical role in the initiation and progress of these diseases.

The IL-1β and IL-18 induced by the inflammasome play a key role to prime the inflammatory response, since they induce the production of many major inflammatory cytokines (TNFα, IL-6, ...), and to activate various cell types involved in the inflammation (such as macrophages, platelets or neutrophils).

The inflammasome-related diseases include, but are not limited to, chronic inflammation, inflammation associated to metabolic disorders (obesity, type 2 diabetes, atherosclerosis), cardiovascular diseases (hypertension, vascular disease/vasoconstriction) and degenerative diseases (Alzheimer's disease, Parkinson's disease).

In an embodiment, the present invention relates to the use as defined above wherein said disease is a chronic inflammation, preferably a low-grade chronic inflammation, or a cardiovascular disease, preferably hypertension.

In an embodiment, the present invention relates to the use as defined above wherein said disease is an inflammation associated to metabolic disorders.

In an embodiment, the present invention relates to the use as defined above wherein said disease is a degenerative disease.

In an embodiment, the present invention relates to the use as defined above wherein said disease is a cardiovascular disease induced by a chronic inflammation.

In the invention, the expression "chronic inflammation" refers to a prolonged and persistent inflammation (for several days, weeks or months) marked chiefly by new connective tissue formation. It can be a continuation of an acute form or a prolonged low-grade form.

In the invention, the expression "low-grade chronic inflammation" is characterized by a prolonged and persistent 2- to 3-fold increase in plasma concentrations of pro-inflammatory cytokines (such as IL-6, TNF-α or IFN-γ) and acute phase proteins (such as C-reactive protein CRP).

In the invention, the expression "cardiovascular disease" refers to any abnormal condition characterized by dysfunction of the heart and blood vessels. It includes, but are not limited to, hypertension, arterial stiffness and stroke.

In an embodiment, the invention relates to the use as defined above, wherein said disease is hypertension.

In an embodiment, the invention relates to the use as defined above, wherein said disease is arterial stiffness.

In an embodiment, the invention relates to the use as defined above, wherein said disease is stroke.

In an embodiment, the invention relates to the use as defined above, wherein said disease is low-grade chronic inflammation and/or hypertension.

In an embodiment, the invention relates to the use as defined above, wherein said at least one nucleotide-derived metabolite is adenine.

In an embodiment, the invention relates to the use as defined above, wherein said at least one nucleotide-derived metabolite is N4-acetylcytidine.

In an embodiment, the invention relates to the use as defined above, wherein said at least one nucleotide-derived metabolite is adenine and N4-acetylcytidine.

As shown by the Inventors, the presence of both N4-acetylcytidine and adenine provide signals for inflammasome activation and secretion of IL1FC. Thus, these two metabolites are preferably used in combination to allow diagnosis of an inflammation-related disease.

In a preferred embodiment, the invention relates to the use as defined above, wherein said subject is a human.

In an embodiment, the invention relates to the use as defined above, wherein said human is at least 60 years old.

In a preferred embodiment, the subject is an older person, preferably over 60, 65, 70, 75, 80, 85, 90, 95 or 100 years old.

In an embodiment, the invention relates to the use as defined above, wherein said sample is blood, serum, plasma or urine, preferably serum.

In an embodiment, the invention relates to the use as defined above, wherein the concentration of said at least one nucleotide-derived metabolite is determined using an assay selected from the group consisting of immunoassays, aptamer-based assays, and mass spectrometry-based assays.

In an embodiment, the invention relates to the use of adenine in the *in vitro*/*ex vivo* diagnosis of a chronic inflammation, in particular low-grade chronic inflammation, in a sample obtained from a subject.

In an embodiment, the invention relates to the use of adenine in the *in vitro*/*ex vivo* diagnosis of a cardiovascular disease, in particular a cardiovascular disease chosen in the group comprising the hypertension, the stroke and the arterial stiffness, in a sample obtained from a subject.

In an embodiment, the invention relates to the use of adenine in the *in vitro*/*ex vivo* diagnosis of inflammation associated to metabolic disorders, in a sample obtained from a subject.

In an embodiment, the invention relates to the use of adenine in the *in vitro*/*ex vivo* diagnosis of a degenerative disease, in a sample obtained from a subject.

In an embodiment, the invention relates to the use of N4-acetylcytidine in the *in vitro*/*ex vivo* diagnosis of a chronic inflammation, in particular a low-grade chronic inflammation, in a sample obtained from a subject.

In an embodiment, the invention relates to the use of N4-acetylcytidine in the *in vitro*/*ex vivo* diagnosis of a cardiovascular disease, in particular a cardiovascular disease chosen in the group comprising the hypertension, the stroke and the arterial stiffness, in a sample obtained from a subject.

In an embodiment, the invention relates to the use of N4-acetylcytidine in the *in vitro*/*ex vivo* diagnosis of inflammation associated to metabolic disorders, in a sample obtained from a subject.

In an embodiment, the invention relates to the use of N4-acetylcytidine in the *in vitro*/*ex vivo* diagnosis of a degenerative disease, in a sample obtained from a subject.

In an embodiment, the invention relates to the use of adenine and N4-acetylcytidine in the *in vitro*/*ex vivo* diagnosis of a chronic inflammation, in particular a low-grade chronic inflammation, in a sample obtained from a subject.

In an embodiment, the invention relates to the use of adenine and N4-acetylcytidine in the *in vitro*/*ex vivo* diagnosis of a cardiovascular disease, in particular a cardiovascular disease chosen in the group comprising the hypertension, the stroke and the arterial stiffness, in a sample obtained from a subject.

In an embodiment, the invention relates to the use of adenine and N4-acetylcytidine in the *in vitro*/*ex vivo* diagnosis of inflammation associated to metabolic disorders, in a sample obtained from a subject.

In an embodiment, the invention relates to the use of adenine and N4-acetylcytidine in the *in vitro*/*ex vivo* diagnosis of a degenerative disease, in a sample obtained from a subject.

In another aspect, the invention also relates to a method for *in vitro*/*ex vivo* diagnosing a disease in a subject, said disease being an inflammation-related disease, said method comprising the step of determining the concentration of at least one nucleotide-derived metabolite selected from the group consisting of adenine and N4-acetylcytidine, in a sample obtained from said subject.

In another aspect, the invention also relates to the method as defined above, wherein said disease is an inflammasome-related disease.

In an embodiment, the present invention relates to the method as defined above wherein said disease is a chronic inflammation, preferably a low-grade chronic inflammation, or a cardiovascular disease, preferably hypertension.

In an embodiment, the present invention relates to the method as defined above wherein said disease is an inflammation associated to metabolic disorders.

In an embodiment, the present invention relates to the method as defined above wherein said disease is a degenerative disease.

In an embodiment, the present invention relates to the method as defined above wherein said disease is a cardiovascular disease induced by a chronic inflammation.

In an embodiment, the invention relates to the method as defined above, wherein said disease is hypertension.

In an embodiment, the invention relates to the method as defined above, wherein said disease is arterial stiffness.

In an embodiment, the invention relates to the method as defined above, wherein said disease is stroke.

In an embodiment, the invention relates to the method as defined above, wherein said disease is low-grade chronic inflammation and/or hypertension.

In an embodiment, the invention relates to the method as defined above, wherein said at least one nucleotide-derived metabolite is adenine.

In an embodiment, the invention relates to the method as defined above, wherein said at least one nucleotide-derived metabolite is N4-acetylcytidine.

In an embodiment, the invention relates to the method as defined above, wherein said at least one nucleotide-derived metabolite is adenine and N4-acetylcytidine.

In an embodiment, the invention relates to the method as defined above, wherein said subject is a human.

In an embodiment, the invention relates to the method as defined above, wherein said human is at least 60 years old.

In an embodiment, the invention relates to the method as defined above, wherein said sample is blood, serum, plasma or urine, preferably serum.

In an embodiment, the invention relates to the method as defined above, wherein the concentration of said at least one nucleotide-derived metabolite is determined using an assay selected from the group consisting of immunoassay, aptamer-based assay, and mass spectrometry-based assay.

In an embodiment, the invention relates to the method as defined above, further comprising a step of comparing the concentration of said at least one nucleotide-derived metabolite in a sample obtained from said subject to a reference value, whereby said disease is to be diagnosed.

In an embodiment, the invention relates to the method as defined above, further comprising a step of determining whether the concentration of said at least one nucleotide-derived metabolite in a sample obtained from said subject is significantly equal to or greater than a minimum concentration of said at least one nucleotide-derived metabolite that is indicative of said disease, preferably, said minimum concentration corresponding to the average concentration of said at least one nucleotide-derived metabolite in samples obtained from subjects with said disease.

In an embodiment, the invention relates to the method as defined above, further comprising a step of determining whether the concentration of said at least one nucleotide-derived metabolite in a sample obtained from said subject is significantly greater than a maximum concentration of said at least one nucleotide-derived metabolite that is indicative of a healthy state, preferably, said maximum concentration corresponding to the average concentration of said at least one nucleotide-derived metabolite in samples obtained from healthy subjects or from subjects with no inflammation-related disease.

In an embodiment, the invention relates to the method as defined above, further comprising a step of determining whether the concentration of N4-acetylcytidine in a serum sample obtained from said subject is greater than 200 nM, preferably greater than 250 nM, more preferably than 300 nM.

In an embodiment, the invention relates to the method as defined above, further comprising a step of determining whether the concentration of adenine in a serum sample obtained from said subject is greater than 100 nM, preferably greater than 150 nM, more preferably than 200 nM.

In the invention, a concentration of N4-acetylcytidine greater than 200 nM in the serum of a subject and/or a concentration of adenine greater than 100 nM in the serum of a subject indicates a risk of an inflammation-related disease, in particular a risk of an inflammasome-related disease, more particularly a risk of chronic inflammation or hypertension.

In an embodiment, the invention relates to a method for *in vitro*/*ex vivo* diagnosing a disease in a subject, said disease being a chronic inflammation, preferably a low-grade chronic inflammation, said method comprising the step of determining the concentration of adenine in a sample obtained from said subject.

In an embodiment, the invention relates to a method for *in vitro*/*ex vivo* diagnosing a disease in a subject, said disease being a cardiovascular disease, preferably chosen in the group comprising the hypertension, the stroke and the arterial stiffness, said method comprising the step of determining the concentration of adenine in a sample obtained from said subject.

In an embodiment, the invention relates to a method for *in vitro*/*ex vivo* diagnosing a disease in a subject, said disease being an inflammation associated to metabolic disorders, said method comprising the step of determining the concentration of adenine in a sample obtained from said subject.

In an embodiment, the invention relates to a method for *in vitro*/*ex vivo* diagnosing a disease in a subject, said disease being a degenerative disease, said method comprising the step of determining the concentration of adenine in a sample obtained from said subject.

In an embodiment, the invention relates to a method for *in vitro*/*ex vivo* diagnosing a disease in a subject, said disease being a chronic inflammation, preferably a low-grade chronic inflammation, said method comprising the step of determining the concentration of N4-acetylcytidine in a sample obtained from said subject.

In an embodiment, the invention relates to a method for *in vitro*/*ex vivo* diagnosing a in a subject, said disease being a cardiovascular disease, preferably chosen in the group comprising the hypertension, the stroke and the arterial stiffness, said method comprising the step of determining the concentration of N4-acetylcytidine in a sample obtained from said subject.

In an embodiment, the invention relates to a method for *in vitro*/*ex vivo* diagnosing a disease in a subject, said disease being an inflammation associated to metabolic disorders, said method comprising the step of determining the concentration of N4-acetylcytidine in a sample obtained from said subject.

In an embodiment, the invention relates to a method for *in vitro*/*ex vivo* diagnosing a disease in a subject, said disease being a degenerative disease, said method comprising the step of determining the concentration of N4-acetylcytidine in a sample obtained from said subject.

In an embodiment, the invention relates to a method for *in vitro*/*ex vivo* diagnosing a disease in a subject, said disease being a chronic inflammation, preferably a low-grade chronic inflammation, said method comprising the step of determining the concentration of adenine and N4-acetylcytidine in a sample obtained from said subject.

In an embodiment, the invention relates to a method for *in vitro*/*ex vivo* diagnosing a disease in a subject, said disease being a cardiovascular disease, preferably chosen in the group comprising the hypertension, the stroke and the arterial stiffness, said method comprising the step of determining the concentration of adenine and N4-acetylcytidine in a sample obtained from said subject.

In an embodiment, the invention relates to a method for *in vitro*/*ex vivo* diagnosing a disease in a subject, said disease being an inflammation associated to metabolic disorders, said method comprising the step of determining the concentration of adenine and N4-acetylcytidine in a sample obtained from said subject.

In an embodiment, the invention relates to a method for *in vitro*/*ex vivo* diagnosing a disease in a subject, said disease being a degenerative disease, said method comprising the step of determining the concentration of adenine and N4-acetylcytidine in a sample obtained from said subject.

In another aspect, the invention also relates to a screening method for determining whether a compound would be effective in the treatment of a disease, said disease being an inflammation-related disease, comprising:
- a step of incubating said compound *in vitro* with cells that produce at least one nucleotide-derived metabolite selected from the group consisting of adenine and N4-acetylcytidine, and
- a step of determining the extent of decrease caused by said compound on the production of said at least one nucleotide-derived metabolite.

In an embodiment, the invention relates to a screening method as defined above, wherein said compound is incubated *in vitro* with cells in the presence of at least one nucleotide-derived metabolite selected from the group consisting of adenine and N4-acetylcytidine.

In an embodiment, the invention relates to a screening method as defined above, further comprising a step of determining the extent of decrease caused by said compound on the IL-1β secretion by said cells.

In an embodiment, the invention relates to a screening method as defined above, further comprising a step of determining the extent of decrease caused by said compound on the IL-18 secretion by said cells.

In a preferred embodiment, cells are chosen among the group comprising: monocytes, macrophages and neutrophils.

In a preferred embodiment, the invention relates to a screening method as defined above, wherein said disease is a chronic inflammation, preferably a low-grade chronic inflammation.

In a preferred embodiment, the invention relates to a screening method as defined above, wherein said disease is a cardiovascular disease, preferably hypertension.

In another aspect, the invention also relates to a screening method for determining whether a compound would be effective in the treatment of a disease, said disease being an inflammation-related disease, comprising:
- a step of administrating said compound to an animal, and
- a step of determining the extent of decrease caused by said compound on the concentration of at least one nucleotide-derived metabolite selected from the group consisting of adenine and N4- acetylcytidine in the serum of said animal.

In an embodiment, the invention relates to a screening method as defined above, wherein said compound is administrated to an animal in the presence of nucleotide-derived metabolite selected from the group consisting of adenine and N4-acetylcytidine. Preferably, said animal is chosen from the group comprising mice, rats and rabbits.

In an embodiment, said animal is not a human.

In an embodiment, the invention relates to a screening method as defined above, further comprising a step of determining the extent of decrease caused by said compound on the concentration of IL-1β in the serum of said animal.

In an embodiment, the invention relates to a screening method as defined above, further comprising a step of determining the extent of decrease caused by said compound on the concentration of IL-18 in the serum of said animal.

In an embodiment, the invention relates to a screening method as defined above, further comprising a step of determining the extent of decrease of blood pressure and/or of arterial stiffness in said animal.

In another aspect, the invention also relates to a screening method for determining whether a compound may induce an inflammatory response:
- a step of incubating said compound *in vitro* with cells that produce at least one nucleotide-derived metabolite selected from the group consisting of adenine and N4-acetylcytidine, and
- a step of determining the extent of increase caused by said compound on the production of said at least one nucleotide-derived metabolite.

In particular, the screening method as defined above can be used for determining whether a compound may induce an inflammatory response involving the inflammasome.

In an embodiment, the invention relates to a screening method as defined above, wherein said compound is incubated *in vitro* with cells in the presence of at least one nucleotide-derived metabolite selected from the group consisting of adenine and N4-acetylcytidine.

In an embodiment, the invention relates to a screening method as defined above, further comprising a step of determining the extent of increase caused by said compound on the IL-1β secretion by said cells.

In an embodiment, the invention relates to a screening method as defined above, further comprising a step of determining the extent of increase caused by said compound on the IL-18 secretion by said cells.

In a preferred embodiment, cells are chosen among the group comprising: monocytes, macrophages and neutrophils.

In another aspect, the invention also relates to a screening method for determining whether a compound may induce an inflammatory response, comprising:
- a step of administrating said compound to an animal, and
- a step of determining the extent of increase caused by said compound on the concentration of at least one nucleotide-derived metabolite selected from the group consisting of adenine and N4-acetylcytidine in the serum of said animal.

In an embodiment, the invention relates to a screening method as defined above, wherein said compound is administrated to an animal in the presence of nucleotide-derived metabolite selected from the group consisting of adenine and N4-acetylcytidine. Preferably, said animal is chosen from the group comprising mice, rats and rabbits.

In an embodiment, said animal is not a human.

In an embodiment, the invention relates to a screening method as defined above, further comprising a step of determining the extent of increase caused by said compound on the concentration of IL-1β in the serum of said animal.

In an embodiment, the invention relates to a screening method as defined above, further comprising a step of determining the extent of increase caused by said compound on the concentration of IL-18 in the serum of said animal.

In an embodiment, the invention relates to a screening method as defined above, further comprising a step of determining the extent of increase of blood pressure and/or of arterial stiffness in said animal.

In another aspect, the invention also relates to a method of monitoring treatment efficacy in a subject undergoing treatment for a disease, said disease being an inflammation-related disease, said method comprising:
- determining the concentration of at least one nucleotide-derived metabolite selected from the group consisting of adenine and N4-acetylcytidine, in samples obtained from said subject over time, and
- determining the evolution of said concentration of at least one nucleotide-derived metabolite, whereby:
   said treatment is effective if said concentration of at least one nucleotide-derived metabolite decreases over time, and
   said treatment is ineffective if said concentration of at least one nucleotide-derived metabolite is stable or increases over time.

In an embodiment, the invention relates to the method of monitoring treatment efficacy as defined above, wherein the concentration of at least one nucleotide-derived metabolite is determined in at least one sample obtained before the treatment and in at least one sample obtained after the first administration of the treatment.

In another aspect, the invention also relates to a kit for diagnosing a disease in a sample, said disease being an inflammation-related disease, comprising:
one or more reagent allowing the detection of at least one nucleotide-derived metabolite selected from the group consisting of adenine and N4-acetylcytidine.

In another aspect, the invention also relates to a kit for monitoring treatment efficacy in a subject undergoing treatment for a disease, said disease being an inflammation-related disease, comprising:
one or more reagent allowing the detection of at least one nucleotide-derived metabolite selected from the group consisting of adenine and N4-acetylcytidine.

In an embodiment, the invention relates to a kit as defined above, wherein said one or more reagent comprises an aptamer for detecting said at least one nucleotide-derived metabolite.

In an embodiment, the invention relates to a kit as defined above, wherein said one or more reagent comprises an aptamer for detecting adenine and/or an aptamer for detecting N4-acetylcytidine.

In a complementary aspect, the invention also relates to the use of the *NLRC4* gene in the *in vitro*/*ex vivo* diagnosis of a cardiovascular disease in a sample obtained from a subject. This invention is based on the unexpected observation made by the Inventors that the *NLRC4* gene is overexpressed in subjects with cardiovascular diseases.

In an embodiment, the invention relates to the use of a nucleic acid, comprising or consisting of a sequence having at least 90% identity with SEQ ID NO: 1, in the *in vitro*/*ex vivo* diagnosis of a cardiovascular disease in a sample obtained from a subject.

In an embodiment, the invention relates to the use of a nucleic acid, comprising or consisting of a sequence having at least 91%, 92%, 93%, 94%, 95%, 96, 97%, 98% or 99% identity with SEQ ID NO: 1, in the *in vitro*/*ex vivo* diagnosis of a cardiovascular disease in a sample obtained from a subject.

In an embodiment, the invention relates to the use of a protein, comprising or consisting of a sequence having at least 90% sequence identity with SEQ ID NO: 2, in the *in vitro*/*ex vivo* diagnosis of a cardiovascular disease in a sample obtained from a subject.

In an embodiment, the invention relates to the use of a protein, comprising or consisting of a sequence having at least 91%, 92%, 93%, 94%, 95%, 96, 97%, 98% or 99% identity with SEQ ID NO: 2, in the *in vitro*/*ex vivo* diagnosis of a cardiovascular disease in a sample obtained from a subject.

**Table 1. Nucleic acid sequence of the NLRC4 gene (Acc. No. AF376061.1) and amino acid sequence of the protein coded by said gene.**

| | |
|---|---|
| **SEQ ID NO: 1** | AGAATGTCATCCTCAAGGGAAGTGCAGAGAGATTTCTTCAGTCCTCAGCTGAGTATA AGCTGGCCTCCTGGAGTCTGTGAACACAAACGTCCAATGTGAGTGTGCCTGTGCAA GCCCCTGGCTGTTTATACTCCGGAGGGTGTCCCCGTGCGTCATCGGTGGAGTGGAC CAAAACTGGTGATCTGTTTGCCCTGTGTGACCTTGCCCAGAACCCTGCTGACTGAGA GAACACATCTGCTGGAAGTCCTCTGGGATTCAAGGTACAGGAAGAACTCGAGGCCT CACTGAAACGGAAAGCAAATACAAAGAAACTTTATTTAAAAACGTGTCTTGGTCTC CCAAGAAGAGGGCAATTGGATTGCTCAGCCAGAATGAAGAGTAGTTTTACAGAAAA AAGAGGACAATATTGGGATCACCTTTGACCTTTCCATTTGGAAATAATATTTTCTATT GTGTTATAGAAAGGTGGGAAGCTTTCATCCAGAACAATGAATTTCATAAAGGACAA TAGCCGAGCCCTTATTCAAAGAATGGGAATGACTGTTATAAAGCAAATCACAGATG ACCTATTTGTATGGAATGTTCTGAATCGCGAAGAAGTAAACATCATTTGCTGCGAGA AGGTGGAGCAGGATGCTGCTAGAGGGATCATTCACATGATTTTGAAAAAGGGTTCA GAGTCCTGTAACCTCTTTCTTAAATCCCTTAAGGAGTGGAACTATCCTCTATTTCAGG ACTTGAATGGACAAAGTCTTTTTCATCAGACATCAGAAGGAGACTTGGACGATTTGG CTCAGGATTTAAAGGACTTGTACCATACCCCATCTTTTCTGAACTTTTATCCCCTTGGT GAAGATATTGACATTATTTTTAACTTGAAAAGCACCTTCACAGAACCAGTCCTGTGG AGGAAGGACCAACACCATCACCGCGTGGAGCAGCTGACCCTGAATGGCCTCCTGCA GGCTCTTCAGAGCCCCTGCATCATTGAAGGGGAATCTGGCAAAGGCAAGTCCACTC TGCTGCAGCGCATTGCCATGCTCTGGGGCTCCGGAAAGTGCAAGGCTCTGACCAAG TTCAAATTCGTCTTCTTCCTCCGTCTCAGCAGGGCCCAGGGTGGACTTTTTGAAACCC |
| | TCTGTGATCAACTCCTGGATATACCTGGCACAATCAGGAAGCAGACATTCATGGCCA TGCTGCTGAAGCTGCGGCAGAGGGTTCTTTTCCTTCTTGATGGCTACAATGAATTCA AGCCCCAGAACTGCCCAGAAATCGAAGCCCTGATAAAGGAAAACCACCGCTTCAAG AACATGGTCATCGTCACCACTACCACTGAGTGCCTGAGGCACATACGGCAGTTTGGT GCCCTGACTGCTGAGGTGGGGATATGACAGAAGACAGCGCCCAGGCTCTCATCCG AGAAGTGCTGATCAAGGAGCTTGCTGAAGGCTTGTTGCTCCAAATTCAGAAATCCA GGTGCTTGAGGAATCTCATGAAGACCCCTCTCTTTGTGGTCATCACTTGTGCAATCC AGATGGGTGAAAGTGAGTTCCACTCTCACACAAACAACGCTGTTCCATACCTTCT ATGATCTGTTGATACAGAAAAACAAACACAAACATAAAGGTGTGGCTGCAAGTGAC TTCATTCGGAGCCTGGACCACTGTGGAGACCTAGCTCTGGAGGGTGTGTTCTCCCAC AAGTTTGATTTCGAACTGCAGGATGTGTCCAGCGTGAATGAGGATGCCCTGCTGAC AACTGGGCTCCTCTGTAAATATACAGCTCAAAGGTTCAAGCCAAAGTATAAATTCTT TCACAAGTCATTCCAGGAGTACACAGCAGGACGAAGACTCAGCAGTTTATTGACGT CTCATGAGCCAGAGGAGGTGACCAAGGGGAATGGTTACTTGCAGAAAATGGTTTCC ATTTCGGACATTACATCCACTTATAGCAGCCTGCTCCGGTACACCTGTGGGTCATCTG TGGAAGCCACCAGGGCTGTTATGAAGCACCTCGCAGCAGTGTATCAACACGGCTGC CTTCTCGGACTTTCCATCGCCAAGAGGCCTCTCTGGAGACAGGAATCTTTGCAAAGT GTGAAAAACACCACTGAGCAAGAAATTCTGAAAGCCATAAACATCAATTCCTTTGTA GAGTGTGGCATCCATTTATATCAAGAGAGTACATCCAAATCAGCCCTGAGCCAAGA ATTTGAAGCTTTCTTTCAAGGTAAAAGCTTATATATCAACTCAGGGAACATCCCCGAT TACTTATTTGACTTCTTTGAACATTTGCCCAATTGTGCAAGTGCCCTGGACTTCATTA AACTGGACTTTTATGGGGGAGCTATGGCTTCATGGGAAAAGGCTGCAGAAGACACA GGTGGAATCCACATGGAAGAGGCCCCAGAAACCTACATTCCCAGCAGGGCTGTATC TTTGTTCTTCAACTGGAAGCAGGAATTCAGGACTCTGGAGGTCACACTCCGGGATTT CAGCAAGTTGAATAAGCAAGATATCAGATATCTGGGGAAAATATTCAGCTCTGCCA CAAGCCTCAGGCTGCAAATAAAGAGATGTGCTGGTGTGGCTGGAAGCCTCAGTTTG GTCCTCAGCACCTGTAAGAACATTTATTCTCTCATGGTGGAAGCCAGTCCCCTCACCA TAGAAGATGAGAGGCACATCACATCTGTAACAAACCTGAAAACCTTGAGTATTCATG ACCTACAGAATCAACGGCTGCCGGGTGGTCTGACTGACAGCTTGGGTAACTTGAAG AACCTTACAAAGCTCATAATGGATAACATAAAGATGAATGAAGAAGATGCTATAAA ACTAGCTGAAGGCCTGAAAAACCTGAAGAAGATGTGTTTATTTCATTTGACCCACTT GTCTGACATTGGAGAGGGAATGGATTACATAGTCAAGTCTCTGTCAAGTGAACCCT GTGACCTTGAAGAAATTCAATTAGTCTCCTGCTGCTTGTCTGCAAATGCAGTGAAAA TCCTAGCTCAGAATCTTCACAATTTGGTCAAACTGAGCATTCTTGATTTATCAGAAAA TTACCTGGAAAAAGATGGAAATGAAGCTCTTCATGAACTGATCGACAGGATGAACG TGCTAGAACAGCTCACCGCACTGATGCTGCCCTGGGGCTGTGACGTGCAAGGCAGC CTGAGCAGCCTGTTGAAACATTTGGAGGAGGTCCCACAACTCGTCAAGCTTGGGTT GAAAAACTGGAGACTCACAGATACAGAGATTAGAATTTTAGGTGCATTTTTTGGAA AGAACCCTCTGAAAAACTTCCAGCAGTTGAATTTGGCGGGAAATCGTGTGAGCAGT GATGGATGGCTTGCCTTCATGGGTGTATTTGAGAATCTTAAGCAATTAGTGTTTTTT GACTTTAGTACTAAAGAATTTCTACCTGATCCAGCATTAGTCAGAAAACTTAGCCAA GTGTTATCCAAGTTAACTTTTCTGCAAGAAGCTAGGCTTGTTGGGTGGCAATTTGAT GATGATGATCTCAGTGTTATTACAGGTGCTTTTAAACTAGTAACTGCTTAAATAAAG TGTACTCGAAG |
| **SEQ ID NO: 2** | MNFIKDNSRALIQRMGMTVIKQITDDLFVWNVLNREEVNIICCEKVEQDAARGIIHMIL KKGSESCNLFLKSLKEWNYPLFQDLNGQSLFHQTSEGDLDDLAQDLKDLYHTPSFLNFYP LGEDIDIIFNLKSTFTEPVLWRKDQHHHRVEQLTLNGLLQALQSPCIIEGESGKGKSTLLQ RIAMLWGSGKCKALTKFKFVFFLRLSRAQGGLFETLCDQLLDIPGTIRKQTFMAMLLKLR QRVLFLLDGYNEFKPQNCPEIEALIKENHRFKNMVIVTTTTECLRHIRQFGALTAEVGDM TEDSAQALIREVLIKELAEGLLLQIQKSRCLRNLMKTPLFVVITCAIQMGESEFHSHTQTTL FHTFYDLLIQKNKHKHKGVAASDFIRSLDHCGDLALEGVFSHKFDFELQDVSSVNEDALL TTGLLCKYTAQRFKPKYKFFHKSFQEYTAGRRLSSLLTSHEPEEVTKGNGYLQKMVSISDI |
| | TSTYSSLLRYTCGSSVEATRAVMKHLAAVYQHGCLLGLSIAKRPLWRQESLQSVKNTTEQ EILKAININSFVECGIHLYQESTSKSALSQEFEAFFQGKSLYINSGNIPDYLFDFFEHLPNCA SALDFIKLDFYGGAMASWEKAAEDTGGIHMEEAPETYIPSRAVSLFFNWKQEFRTLEVT LRDFSKLNKQDIRYLGKIFSSATSLRLQIKRCAGVAGSLSLVLSTCKNIYSLMVEASPLTIED ERHITSVTNLKTLSIHDLQNQRLPGGLTDSLGNLKNLTKLIMDNIKMNEEDAIKLAEGLK NLKKMCLFHLTHLSDIGEGMDYIVKSLSSEPCDLEEIQLVSCCLSANAVKILAQNLHNLVK LSILDLSENYLEKDGNEALHELIDRMNVLEQLTALMLPWGCDVQGSLSSLLKHLEEVPQL VKLGLKNWRLTDTEIRILGAFFGKNPLKNFQQLNLAGNRVSSDGWLAFMGVFENLKQL VFFDFSTKEFLPDPALVRKLSQVLSKLTFLQEARLVGWQFDDDDLSVITGAFKLVTA |

In an embodiment, the invention relates to the use as defined above, wherein said cardiovascular disease is hypertension and/or arterial stiffness.

In an embodiment, the invention relates to the use as defined above, wherein said subject is a human.

In an embodiment, the invention relates to the use as defined above, wherein said human is at least 60 years old.

In a preferred embodiment, the subject is an older person, preferably over 60, 65, 70, 75, 80, 85, 90, 95 or 100 years old.

In an embodiment, the invention relates to the use as defined above, wherein said sample is blood, serum, plasma, urine or PBMC (*Peripheral Blood Mononuclear Cells*), preferably serum.

In an embodiment, the invention relates to the use as defined above, wherein the expression of said *NLRC4* gene is determined by measuring the concentration of mRNA thereof using an assay selected from the group consisting of RT-PCR, microarray or northern blot.

In an embodiment, the invention relates to the use as defined above, wherein the expression of said *NLRC4* gene is determined by measuring the concentration of the protein thereof using an immunoassay such as immunoblot or ELISA.

In another aspect, the invention also relates to a method for *in vitro*/*ex vivo* diagnosing a cardiovascular disease in a subject, said method comprising the step of determining the expression of the *NLRC4* gene, in a sample obtained from said subject.

In an embodiment, the invention relates to the method as defined above, further comprising a step of determining whether the expression of the *NLRC4* gene in a sample obtained from said subject is significantly equal to or greater than a minimum expression of the *NLRC4* gene that is indicative of a cardiovascular disease, preferably said minimum expression corresponding to the average expression of the *NLRC4* gene in samples obtained from subjects with said disease.

In an embodiment, the invention relates to the method as defined above, further comprising a step of determining whether the expression of the *NLRC4* gene in a sample obtained from said subject is significantly greater than a maximum expression of the *NLRC4* gene that is indicative of a healthy state, preferably said maximum expression corresponding to the average expression of the *NLRC4* gene in samples obtained from healthy subjects or from subjects with no inflammation-related disease.

In an embodiment, the invention relates to the method as defined above, further comprising a step of determining whether the expression of the *NLRC4* gene in a sample obtained from said subject is at least 1.5 times higher than the average expression of the *NLRC4* gene in samples obtained from healthy subjects or from subjects with no inflammation-related disease.

In the invention, a 1.5 fold increase of the expression of the *NLRC4* gene indicates a risk of cardiovascular disease, in particular hypertension.

In another aspect, the invention also relates to a screening method for determining whether a compound would be effective in the treatment of a cardiovascular disease, preferably hypertension, comprising:
- a step of incubating said compound *in vitro* with cells expressing the *NLRC4* gene, and
- a step of determining the extent of decrease caused by said compound on the expression of the *NLRC4* gene.

In an embodiment, the invention relates to a screening method as defined above, further comprising a step of determining the extent of decrease caused by said compound on the IL-1β secretion by said cells.

In an embodiment, the invention relates to a screening method as defined above, further comprising a step of determining the extent of decrease caused by said compound on the IL-18 secretion by said cells.

In a preferred embodiment, cells are chosen among the group comprising: monocytes, macrophages and neutrophils.

In another aspect, the invention also relates to a screening method for determining whether a compound would be effective in the treatment of a cardiovascular disease, preferably hypertension, comprising:
- a step of administrating said compound to an animal, and
- a step of determining the extent of decrease caused by said compound on the expression of the *NLRC4* gene in blood cells of said animal (at least monocytes). Preferably, said animal is chosen from the group comprising mice, rats and rabbits.

In an embodiment, said animal is not a human.

In an embodiment, the invention relates to a screening method as defined above, further comprising a step of determining the extent of decrease caused by said compound on the concentration of IL-1β in the serum of said animal.

In an embodiment, the invention relates to a screening method as defined above, further comprising a step of determining the extent of decrease caused by said compound on the concentration of IL-18 in the serum of said animal.

In an embodiment, the invention relates to a screening method as defined above, further comprising a step of determining the extent of decrease of blood pressure and/or of arterial stiffness in said animal.

In another aspect, the invention also relates to a screening method for determining whether a compound may induce hypertension:
- a step of incubating said compound *in vitro* with cells expressing the *NLRC4* gene,and
- a step of determining the extent of increase caused by said compound on the expression of the *NLRC4* gene.

In another aspect, the invention also relates to a screening method for determining whether a compound may induce hypertension comprising:
- a step of administrating said compound to an animal, and
- a step of determining the extent of increase caused by said compound on the expression of the *NLRC4* gene in blood cells of said animal (at least monocytes).

In another aspect, the invention also relates to a method of monitoring treatment efficacy in a subject undergoing treatment for a cardiovascular disease, said method comprising:
- determining the expression of the *NLRC4* gene in samples obtained from said subject over time, and
- determining the evolution of the expression of the *NLRC4* gene, whereby:
   said treatment is effective if said expression of the *NLRC4* gene decreases over time, and
   said treatment is ineffective if said expression of the *NLRC4* gene is stable or increases over time.

In an embodiment, the invention relates to the method of monitoring treatment efficacy as defined above, wherein the expression of the *NLRC4* gene is determined in at least one sample obtained before the treatment and in at least one sample obtained after the first administration of the treatment.

In another aspect, the invention also relates to a kit for diagnosing a cardiovascular disease in a sample, comprising one or more reagent allowing the measurement of the expression of the *NLRC4* gene.

The invention is illustrated by the following figures and examples. These examples are not intended to be limitations of the invention.

### FIGURES

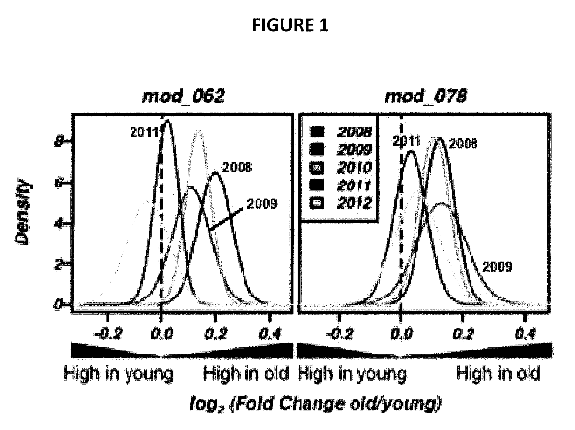

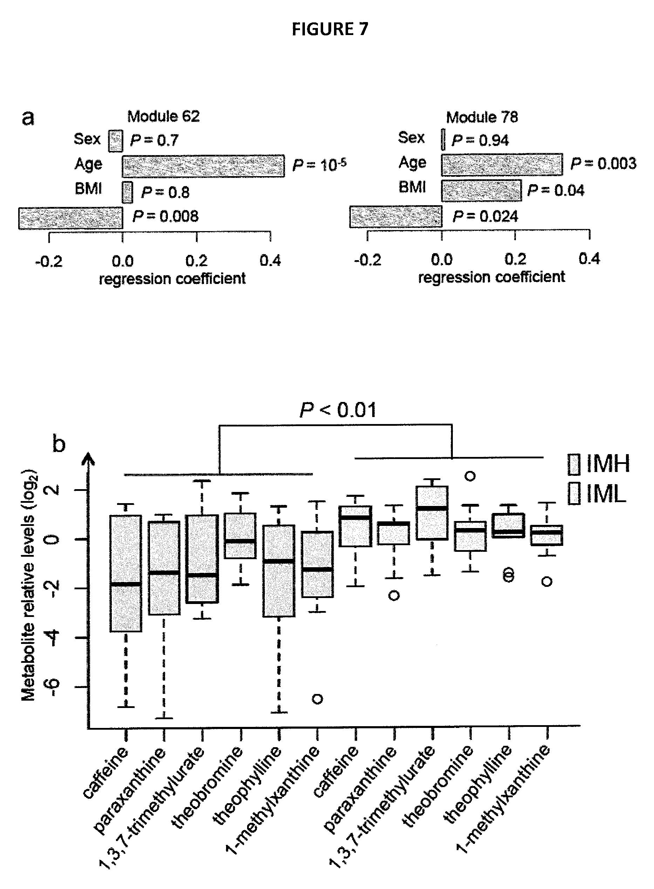

**Figure 1****. High expression of inflammasome gene modules in older adults is longitudinally stable.** Gene expression data from the Stanford-Ellison study (N = 89) was used to find age-associated gene modules that participate in cytokine production and enriched for inflammasome genes (see also Figure 7). For the determination of significant differences in the expression of inflammasome gene modules 62 and 78, the QuSAGE gene set analysis method was used. Positive fold change values (x-axis) indicate higher expression in aged individuals. P-value for age on combined data for each gene module <10⁻³.

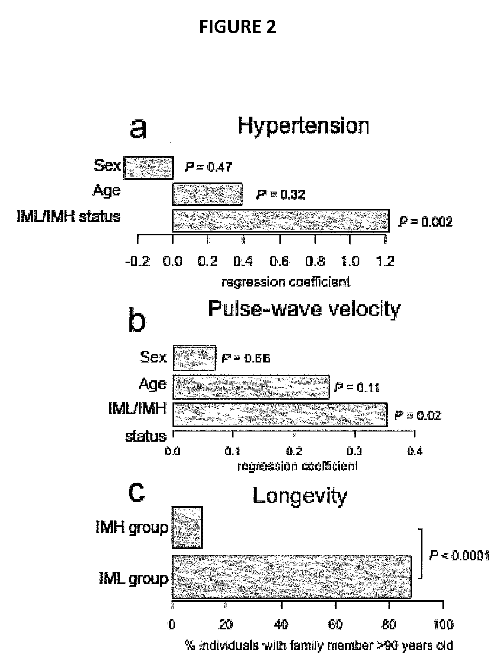

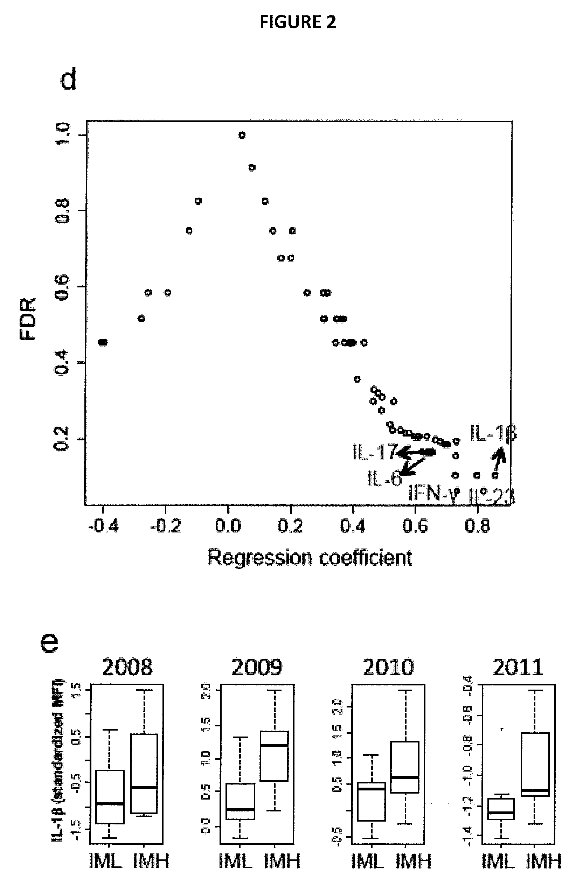

**Figure 2****. Inflammasome module high (IMH) older subjects exhibit high rates of hypertension, elevated arterial stiffness, poor history of familial longevity and signs of chronic inflammation.** Logistic regression analysis was conducted on IML (N = 11) and IMH (N = 12) categories and hypertension (shown are regression coefficients for age, sex and IML/IMH status) (a). A follow-up study consisting in a total of 17 extreme phenotypes shows a significant association of IML versus IMH categories with arterial stiffness as measured by pulse-wave velocity (b). Familial longevity as determined by belonging to a family with at least one family member over 90 years of age is more frequent in IML than in IMH (c). Serum levels of 62 different cytokines, chemokines and growth factors were compared between IML and IM subjects using data from year 2013 (IML *N* = 8, IMH *N =* 8). (d) Multiple regression analysis on each analyte's MFI against age, sex and IML/IMH status was conducted (adjusted for covariates) and significance (y-axis) was obtained via permutation tests. The largest difference is observed for IL-1β, which is stably increased in the IMH group, as shown by longitudinal analysis of data collected during the years 2008-2011 (IML *N* (2008, 2009, 2010, 2011) = 10, 10, 8, 7, respectively and IMH *N* (2008, 2009, 2010, 2011) = 12, 11, 12, 8, respectively) (e).

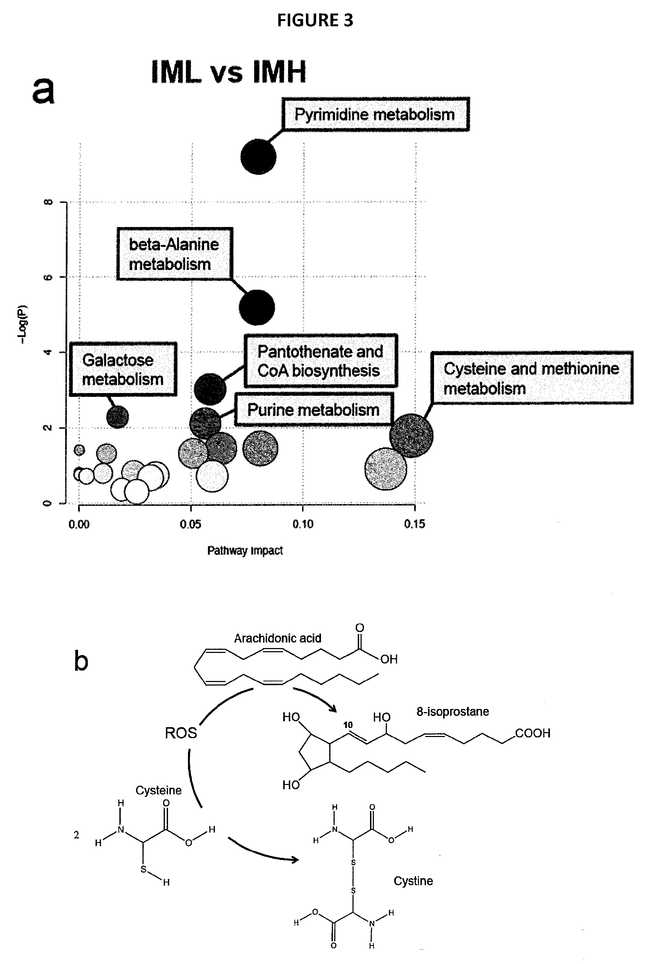

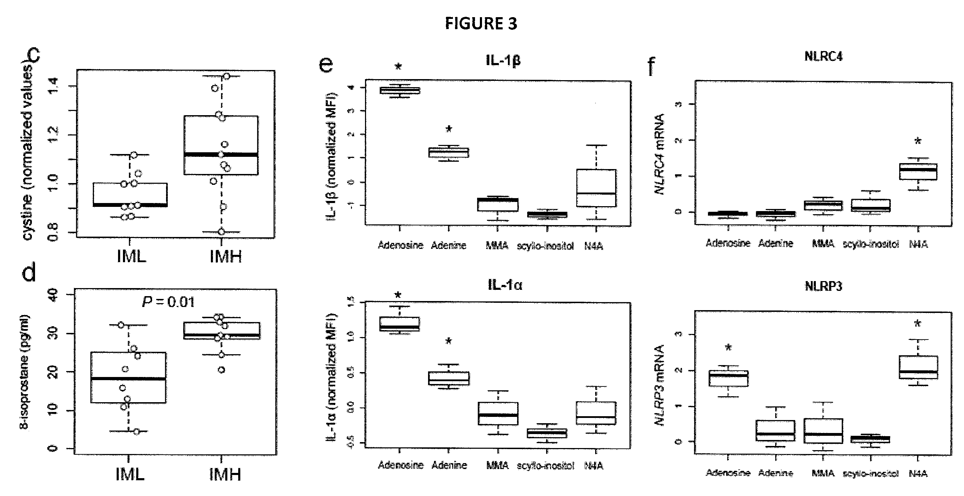

**Figure 3****. High levels of oxidative stress and increased nucleotide metabolites in IMH individuals can induce cytokine production and expression of inflammasome genes in primary monocytes.** Broad-coverage metabolomics profiling was conducted on available serum samples from year 2011 (9 IML and 11 IMH individuals). From a total of 692 metabolites analyzed, 67 were differentially expressed (all up-regulated) in IMH versus IML at an FDR < 0.2 (by SAM analysis). Functional annotation and pathway analysis was conducted using MetPA. A significant enrichment for several metabolic pathways was identified for these metabolites (P < 0.05) (a). Conversion of cysteine to cystine; and from arachidonic acid to 8-isoprostane, in the presence of ROS (b). Increased circulating levels of cystine and 8-isoprostane in IMH (b and c) indicate higher levels of oxidative stress (c and d). Adenine, DL-4-hydroxy-3-methoxymandelic acid (vanillylmandelate) (MMA), scyllo-inositol and N4-acetylcytidine (N4A) were selected based on their levels of significance (*Q* < 0.001, see Table 5) and representing different metabolic pathways, to assess their ability to induce IL1FC and expression of *NLRC4* in primary monocytes from four healthy donors (shown are the results of one representative experiment). Adenosine was used as a positive control. A significant induction of IL1FC is observed for adenosine and adenine, but not with other compounds (e). The highest dose of each compound (100 µM) was used to determine expression of *NLRC4* and *NLRP3* by qPCR on the same samples used for cytokine determination (e). A significant increase in *NLRC4* and *NLRP3* is shown only for N4-acetylcytidine (*P* < 0.01). As previously shown in mice, adenosine treatment up-regulates *NLRP3* gene expression (*P* < 0.01) (f). Expression of GAPDH was used to standardize the samples, and the results are expressed as a ratio relative to control. Error bars reflect experimental variability.

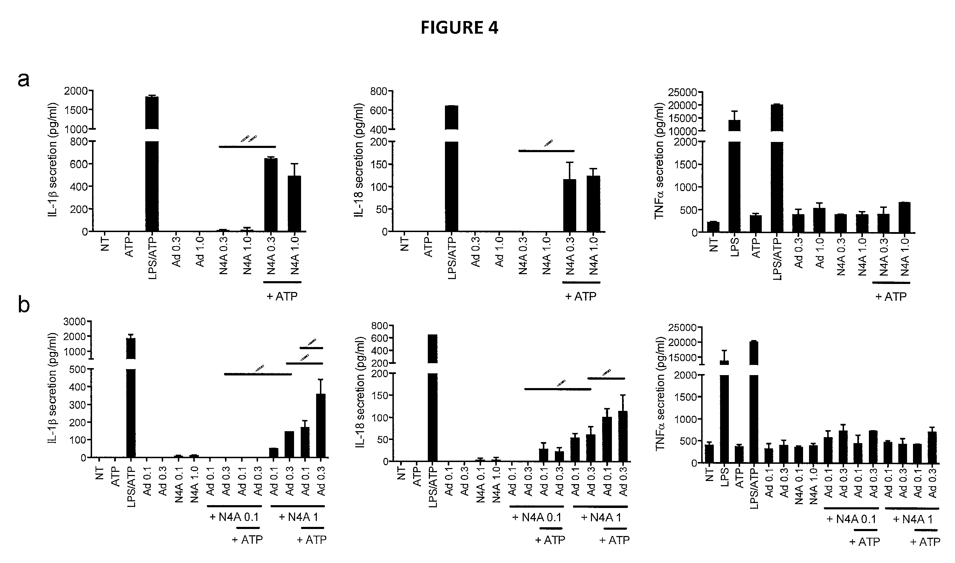

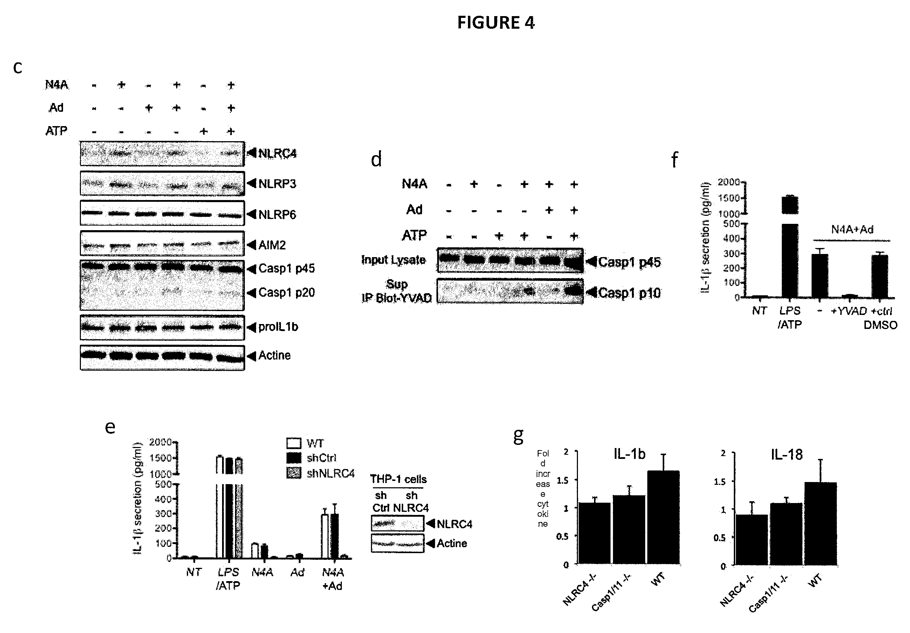

**Figure 4****. N4-Acetylcytidine and Adenine induce the NLRC4 inflammasome.** (a) Differentiated THP-1 cells were treated with ATP (5 mM, 30 min), or primed with LPS (1 µg/ml, 4 hr) and then pulsed with ATP; or treated with indicated concentrations (mM) of Adenine (Ad) or N4-Acetylcytidine (N4A) alone for 6 hr or (b) in combination, and then pulsed or not with ATP. Secretion of cytokines IL-1β, IL-18 and TNF-α were measured by ELISA from cell culture supernatants. (c) Differentiated THP-1 cells were treated with compounds as indicated (1mM N4A; 300 µM Ad) for 6 hr or ATP 5 mM 30 min, cells were lysed and cell lysates were then immunoblotted with various antibodies to monitor expression of NLRs, Casp1, and proIL-1β. (d) Differentiated THP-1 cells were treated with compounds as before and cell lysates were submitted to immunoprecipitation with Biotinyl-YVAD-fmk peptide. Complexes were then recovered by using Streptavidinsepharose beads and immunoblotted with anti-Caspase-1 p10 antibody. (e) Differentiated THP-1 cells were treated with compounds as before in the presence or not of Ac-YVAD-fmk or control DMSO, and IL-1β secretion was measured. (f) Differentiated WT or stable shTHP-1 cell lines were treated with compounds as before (1mM N4A; 300 µM Ad) and IL-1β secretion was measured. To the right, western-blots showing protein expression of NLRC4 in stable shTHP-1 cell lines. (g) Bone marrow-derived macrophages from WT or KO mice as indicated were isolated and treated with combination of compounds (1mM N4A; 300 µM Ad), and IL-1β secretion was measured. Data are expressed as concentration of cytokines (pg/ml) (mean ± SD; *n* = 3). ^{★} *P* < 0.05, ^{★★} *P* < 0.01.

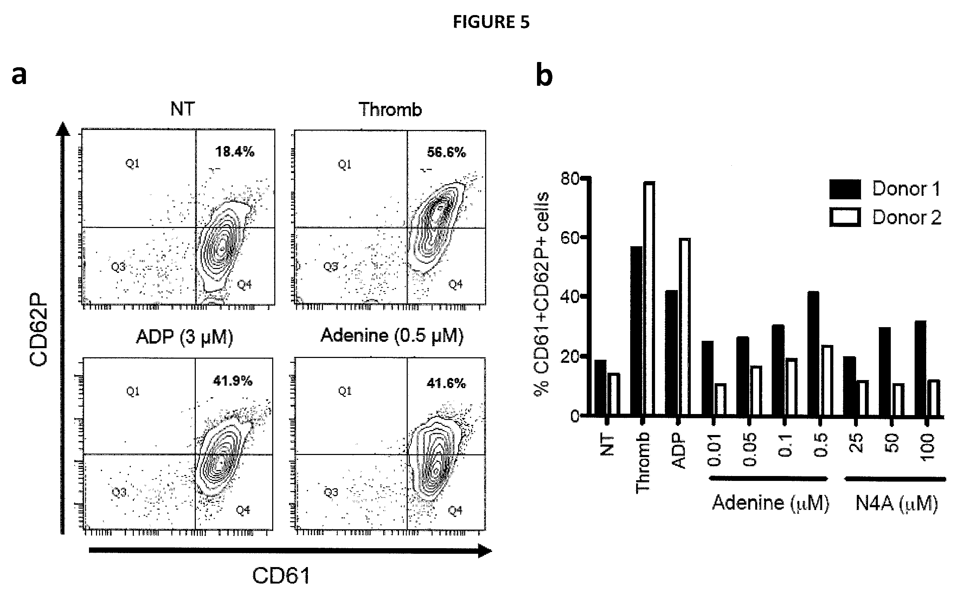

**Figure 5****. N4**-**Acetylcytidine and Adenine activate platelets.** Primary platelets were isolated from the blood of two donors and incubated for 6 hr at 37°C with thrombin, or ADP, or various concentrations of Adenine or N4-Acetylcytidine. Platelet activation was monitored by measuring membrane expression of CD61 and CD62P by flow cytometry. Data are representative of at least three independent experiments with similar results.

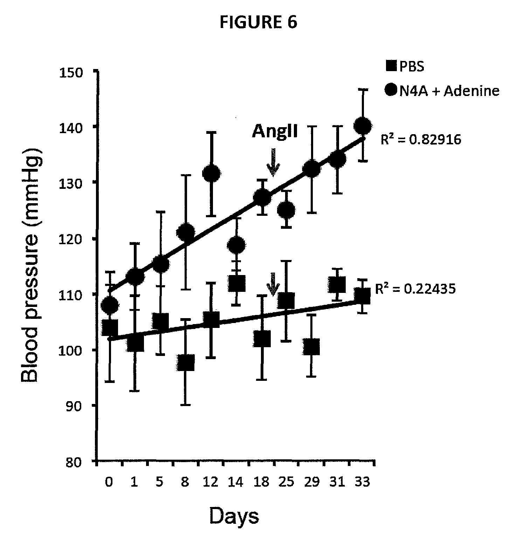

**Figure 6****. Nucleotide metabolites identified in IMH older adults induce high blood pressure in mice.** Adult mice (12-18-week old) were injected mice with N4A plus adenine at 20 mM stock solution 100 ul/25 g body weight once daily, and changes in blood pressure were monitored using a tail cuff method. Treatment with N4A and adenine had a mild effect with a borderline significant increase in blood pressure (prehypertension) as early as 8 days after the first injection (P = 0.02). At day 20 angiotensin II-containing osmotic pumps (at 140 ng/kg/min) in combination with Ad and N4A (in the compound-treated mice) or in combination with PBS (for the control mice) were implanted subcutaneously. Significant increases were observed in the treated mice with an average systolic blood pressure of 140 (±7) compared with 112 (±3) mmHg in the control group (P = 0.008).
**Figure 7****. Caffeine negatively correlates with expression of inflammasome gene modules 62 and 78 and coffee-derived metabolites are increased in IML subjects**. Multiple regression analysis was conducted on expression levels of module 62 and 78 (from data collected in year 2008, *N* = 89) and caffeine intake in mg/week (adjusted for age, sex and BMI). A significant association was found between caffeine intake and expression of modules 62 (*P* < 0.01) and 78 (*P* = 0.024) (a). Differences in circulating levels of coffee and coffee-derived metabolites between were computed using one-tail student's t-test and *P-*values were combined using Fisher's combined probability (*P* < 0.01) (b).

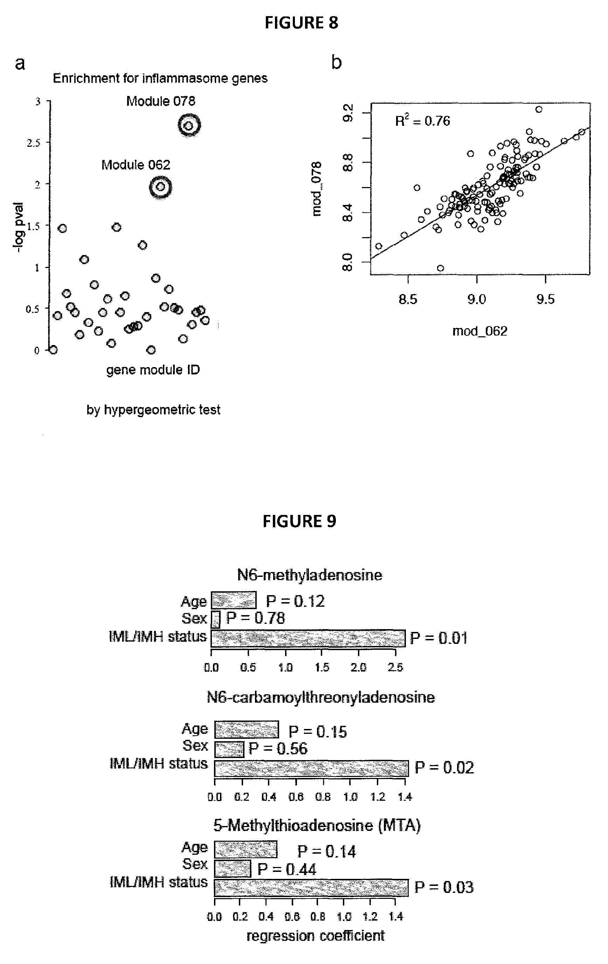

**Figure 8****. Modules 62 and 78 are enriched for inflammasome genes and their expression levels are highly correlated.** (a) Enrichment analysis was conducted for 41 age-associated gene modules derived from our previous studies of aging by hypergeometric test. Both gene modules 62 and 78 were significantly enriched for inflammasome genes (*P* < 0.01). Gene expression of modules 62 and 78 was highly correlated across 89 individuals from the year 2008 data set (*P* < 0.01) (b).
**Figure 9****. Adenosine derivatives are increased in IMH compared to IML subjects.** The levels of adenosine and adenosine derivatives including N1-methyladenosine, N6-methyladenosine, N6-carbamoylthreonyladenosine, N6-succinyladenosine and 5-methylthioadenosine were compared between IML and IMH groups my multiple regression (adjusted for age and sex). Significant differences were found for N6-methyladenosine, N6-carbamoylthreonyladenosine 5-methylthioadenosine (P < 0.05). The bars represent the magnitude of regression coefficient from the fits.

### EXAMPLES

### Example 1. Higher expression of selective inflammasome gene modules in older adults

To investigate changes in the expression of genes from immune cells in human aging, the presence of age-related genes was analyzed in the Stanford-Ellison longitudinal cohort using a modular approach for gene expression data. A gene module is defined as a set of co-expressed genes under the control of common transcription factors likely acting as regulatory programs. An important feature of this approach is that genes, regardless of their functional annotation, are organized into modules based on coordinated expression of their components; such modules may contain genes previously known to be involved in a function and those whose function is yet to be discovered. Using this approach, it was found that of a total 109 gene modules derived from data collected during the year 2008 were correlated with age (FDR *Q* ≤ 0.05) of which only two (modules 62 and 78, composed of 82 and 17 genes, respectively) were annotated to participate in cytokine production based on gene functional annotation analysis (*P* < 0.01). To confirm in an unbiased fashion that from the 41 age-associated gene modules only these gene modules were enriched for inflammasome-connected genes, hypergeometric tests were conducted and it was found significant enrichment for only module 62 and 78 (FDR *Q* < 0.01) (Figure 8a).

The module 78 contained *NLRC4* and module 62 contained *NLRC5* and *IL1B* among other genes related to inflammasome activity such as *IL1RN, TLR6* and *TLR8* (module 62); and *IFAR1* and *TLR5* (module 78). The module 62 was also annotated to participate in cell death (*P* < 0.05), which was not surprising given that activation of inflammatory caspases may lead to rapid pyroptotic cell death besides cytokine maturation. Interestingly, these two gene modules appear to be controlled by similar transcription factors. For example, the genes BCL6, CEBPB, ETS2, MXD4 and NFIL3 were present in the regulatory programs of both gene modules (enrichment *P* <0.01).

To determine the stability of the age-associations for module 62 and 78, we analyzed data from samples collected over five consecutive years (2008-2012) in the Stanford-Ellison cohort. Each year consisted of both new subjects and subjects from previous years who were able to return (Table 2), and the expression of the gene modules 62 and 78 in young versus older subjects was compared using the QuSAGE gene set analysis method.

**Table 2. Number of young (20-30 years) and older (60- >89 years) individuals per year.**

| | 2008 | 2009 | 2010 | 2011 | 2012 |
|---|---|---|---|---|---|
| Young | 29 | 22 | 20 | 28 | 19 |
| Old | 60 | 51 | 55 | 59 | 52 |

For this analysis, samples from the individuals' first appearance in the study (*N* = 114) were used to analyze the age associations for module expression. When considered together, these datasets show a significant age-dependent increase in baseline levels for both gene modules (Figure 1, *P* < 10-3). These results demonstrate that, at the population level, genes in the inflammasome pathway are up-regulated in human aging and that these changes are longitudinally stable.

### Example 2. Persistent expression of inflammasome modules 62 and 78 correlates with hypertension, central arterial stiffness and self-reported familial longevity

Because chronic inflammation has been linked to various age-associated diseases, it was investigated whether the expression of modules 62 and 78 was associated with clinical phenotypes in the aging cohorts. To do so, extreme phenotypes were defined using a classification based both on the magnitude and the chronicity in the expression levels of modules 62 and 78. Subjects were assigned into inflammasome module high (IMH) or inflammasome module low (IML) groups if they were in the upper or lower quartiles, respectively, for each gene module in 3 or more of the 5 years analyzed. Subjects who were not in either of these categories were not included in the analysis. For module 62, this analysis yielded 19 individuals with extreme phenotypes: 9 IMH and 10 IML individuals, and for module 78, 16 individuals: 9 IMH and 7 IML. It was noted a significant degree of overlap for modules 62 and 78 on each category (6 IMH and 6 IML, P-value for enrichment < 0.001). Furthermore, expression levels of these two genes modules across all individuals were highly correlated (R2 = 0.76, *P* < 10-5) (Figure 8b).

Thus, to improve statistical power, IMH or IML individuals from modules 62 and 78 were combined (*N* = 23) for further analysis. A logistic regression analysis was conducted to compare the IMH and IML phenotypes with respect to their clinical history of diabetes, hypertension and psychiatric disorders. No significant associations were found for diabetes or psychiatric disorders. However, it was found that 75% (9/12) of IMH subjects were hypertensive (essential hypertension) compared to almost none (1/11 or 9%) in the IML group. The hypertension rate for all the individuals in the older cohort (ages 60 to >89) was 52%, compared to 65% in people over 60 years old in the US20. Because the age range of our older cohorts was relatively large (60 - >89), age and sex were included in the logistic regression models. In addition, the analysis was adjusted for other confounding factors such as medication history (Table 3) and body mass index (BMI) (see Methods), and it was still found a significant association between hypertension and IMH/IML status (*P* = 0.002) (Figure 2a).

**Table 3. List of medications prescribed in IML and IMH subjects.**

| ***Name*** | ***Mechanism of action*** | ***Class*** |
|---|---|---|
| Amlodipine | Calcium channel blocker | 1 |
| Atacand | Angiotensin II receptor antagonist | 2 |
| Atenolol | Beta-blocker | 3 |
| Benicar | Angiotensin II receptor antagonist | 2 |
| Candesartan | Angiotensin II receptor antagonist | 2 |
| Cartia XT | Calcium channel blocker | 1 |
| Carvedilol | Beta-blocker | 3 |
| Chlorthalidone | Thiazide diuretic | 4 |
| Diltiazem (also XR version) | Calcium channel blocker | 1 |
| Diovan | Angiotensin II receptor antagonist | 2 |
| Doxazosin | Alpha-adrenergic blocker | 5 |
| Enalapril | ACE inhibitor | 6 |
| Furosemide (AKA Lasix) | Loop diuretic | 7 |
| HCTZ (hydrochlorothiazide) | Thiazide diuretic | 4 |
| Lisinopril | ACE inhibitor | 6 |
| Lisinopril+HCTZ | ACE inhibitor+thiazide diuretic | 6*4 |
| Metoprolol | Beta-blocker | 3 |
| Spironolactone | Potassium-sparing diuretic | 8 |
| Triamterene | Potassium-sparing diuretic | 8 |

Based on the observation that the IMH and IML groups differed in their history of diagnosed hypertension and the potential contribution of other confounders, a follow-up study was conducted to determine arterial stiffness (a stable risk factor for cardiovascular complications) using carotid-femoral pulse wave velocity (PWV) testing. The PWV, a measure of central arterial stiffness, was significantly lower in the IML group (7.9 ± 2.4 m/s) compared to the IMH group (10.7 ± 2.1 m/s) (*P* = 0.02) (Figure 2b).

Interestingly, self-reported familial longevity, as determined by belonging to a family with at least one family member over 90 years of age, was significantly higher in IML subjects (88%) compared with IMH ones (11%) *(P* < 10-4) (Figure 2c). Thus, a cluster of unsuccessful aging-related clinical phenotypes including hypertension, arterial stiffness and poor history of familial longevity are represented in the IMH extreme phenotype. These results provide a mechanistic link explaining previous clinical observations showing that (i) the risk of developing hypertension is significantly lower in long-lived families and (ii) arterial stiffness predicts cardiovascular disease and stroke independent of age, gender and blood pressure.

### Example 3. IMH older individuals are chronically inflamed with high levels of circulating lL1FC

The role of chronic inflammation in hypertension and vascular remodeling has gained increasing attention in the past decade and most studies have focused on important mediators of chronic inflammation such as the levels of inflammatory markers such as CRP, IL-6, TNF-α, and IL-1β. Thus, a comparison of circulating levels of a total of 62 cytokines and chemokines obtained from serum samples collected in the same individuals during their yearly visit in 2013 was conducted. A regression analysis on each cytokine against the IML vs IMH status was conducted and adjusted for age and sex. To obtain significance for the regression coefficients, we performed resampling analysis over 500 permutations. We found a significant increase in 17 cytokines (FDR *Q* < 0.2) (Table 4), among which IL-1β, IL-6, IL-23, IFN-γ and IL-17 (Figure 2d) were also recently identified in an inflammatory model of hypertension in mice.

**Table 4. List of cytokines and chemokines associated with the IMH vs IML groups of older individuals.**

| **analyte** | **intercept** | **IMH_IML** | **Sex** | **Age** |
|---|---|---|---|---|
| TGFA | 0.07153846 | 0.062 | 0.744 | 0.868 |
| NGF | 1 | 0.062 | 0.744 | 0.8266667 |
| IL1B | 0.07153846 | 0.1033333 | 0.93 | 0.6763636 |
| IL31 | 0.775 | 0.1033333 | 0.93 | 1 |
| IL23 | 1 | 0.1033333 | 0.5425 | 1 |
| IFNB | 0.07294118 | 0.155 | 0.9358491 | 1 |
| IL17F | 1 | 0.1641176 | 0.9789474 | 1 |
| IL21 | 0.08611111 | 0.1641176 | 0.7560976 | 0.868 |
| 5DF1A | 0.07294118 | 0.1653333 | 0.7294118 | 1 |
| IL6 | 1 | 0.1653333 | 0.7294118 | 1 |
| MCSF | 0.10333333 | 0.1653333 | 0.62 | 1 |
| FGFB | 0.062 | 0.1653333 | 0.7294118 | 1 |
| IL12P70 | 0.10333333 | 0.186 | 0.7294118 | 1 |
| GROA | 0.062 | 0.186 | 0.62 | 1 |
| IFNG | 0.093 | 0.19375 | 0.7294118 | 0.6676923 |
| MIG | 0.18083333 | 0.19375 | 0.744 | 1 |
| IL13 | 0.07153846 | 0.1972727 | 0.8414286 | 0.6716667 |
| IL27 | 0.10333333 | 0.2066667 | 0.8266667 | 1 |
| PIGF1 | 0.07153846 | 0.2066667 | 0.8414286 | 0.6888889 |
| TRAIL | 0.07294118 | 0.2066667 | 0.7294118 | 1 |
| IL1A | 0.07153846 | 0.2066661 | 0.8414286 | 0.6888889 |
| TNFA | 1 | 0.2156522 | 0.744 | 1 |
| CD40L | 1 | 0.2156522 | 0.7560976 | 1 |
| IL8 | 0.07153846 | 0.2232 | 0.62 | 0.682 |
| MIP1B | 1 | 0.2232 | 0.7294118 | 0.7971429 |
| MIP1A | 0.07294118 | 0.2384615 | 0.8857143 | 1 |
| TNFB | 1 | 0.2755556 | 0.9581818 | 1 |
| IL4 | 0.12681818 | 0.2993103 | 0.9358491 | 1 |
| GCSF | 1 | 0.2993103 | 0.7560976 | 1 |
| IL15 | 0.093 | 0.31 | 0.8266667 | 1 |
| IL2 | 1 | 0.32 | 0.9789474 | 1 |
| UF | 1 | 0.329375 | 0.8857143 | 1 |
| FASI | 0.07153846 | 0.3569697 | 0.7294118 | 1 |
| IL5 | 0.2325 | 0.4536585 | 1 | 1 |
| EOTAXIN | 0.63599744 | 0.4536585 | 0.8857143 | 1 |
| SCF | 0.19076923 | 0.4536585 | 1 | 1 |
| IFNA | 0.07153846 | 0.4536585 | 0.7560976 | 1 |
| TGFB | 0.22 | 0.4536585 | 0.9358491 | 1 |
| MCP1 | 0.10333333 | 0.4536585 | 0.7294118 | 1 |
| EGF | 0.24424242 | 0.4536585 | 0.8266667 | 1 |
| MCP3 | 1 | 0.4536585 | 0.7560976 | 1 |
| IL10 | 1 | 0.5166667 | 1 | 1 |
| IL17A | 0.22 | 0.5166667 | 0.9789474 | 1 |
| IL1RA | 1 | 0.5166667 | 0.744 | 1 |
| GMCSF | 1 | 0.5166667 | 0.744 | 1 |
| VEGFD | 0.27352941 | 0.5166667 | 0.7560976 | 1 |
| RESISTIN | 0.16173913 | 0.5166667 | 0.744 | 1 |
| VEGF | 1 | 0.5166667 | 0.8266667 | 1 |
| BONF | 1 | 0.5849057 | 0.6888889 | 1 |
| IL18 | 0.22962963 | 0.5849057 | 0.8857143 | 1 |
| VCAM1 | 0.22 | 0.5849057 | 1 | 1 |
| IL22 | 1 | 0.5849057 | 0.775 | 1 |
| LEPTIN | 1 | 0.5849057 | 0.744 | 1 |
| RANTES | 0.90731707 | 0.6763636 | 0.5425 | 1 |
| HGF | 1 | 0.6763636 | 0.9789474 | 1 |
| IL7 | 1 | 0.7482759 | 0.9358491 | 1 |
| I19 | 0.19076923 | 0.7482759 | 0.5166667 | 1 |
| PAI1 | 0.63589744 | 0.7482759 | 0.7294118 | 1 |
| IL12P40 | 0.63589744 | 0.8266667 | 1 | 1 |
| ICAM1 | 0.53142857 | 0.8266667 | 0.7294118 | 1 |
| IP10 | 0.22 | 0.9147541 | 0.7294118 | 1 |
| PDGFBB | 0.63589744 | 1 | 0.7560976 | 1 |

The largest differences were observed for IL-1β, which was stably increased in the IMH group as shown by longitudinal analysis of data collected during the years 2008-2011 (Figure 2e).

These results demonstrate that a state of immune activation with constitutive production of IL1FC and other inflammatory cytokines characterizes subjects in the IMH group compared to the IML group, which is also in agreement with two recent reports showing that gain-of-function mutations on *NLRC4* cause a macrophage activation syndrome with constitutive IL-1β production.

### Example 4. Nucleotide metabolism dysfunction and oxidative stress in IMH older adults

Maturation and release of IL1FC are controlled by the inflammasome machinery. Core components of this machinery are regulated at transcriptional (priming) and posttranscriptional (activation) levels following a plethora of inflammatory stimuli including metabolites. Given the accumulating evidence suggesting metabolic control in both steps, it was hypothesized that metabolic dysfunction may lead to the generation of potential circulating danger-associated molecular patterns (DAMPs) that trigger expression of inflammasome genes and increase production of inflammatory cytokines observed in the IMH group. To test this, a metabolomic-profiling analysis was conducted across a total of 692 metabolites that were quantified from available sera of 11 IML and 9 IMH individuals by mass spectrometry. To search for significant differences between the two groups, significance analysis of microarray (SAM) analysis was conducted. A total of 67 metabolites were significantly different between the two groups (FDR *Q* < 0.2), all up-regulated in the IMH compared to the IML group (Table 5). Pathway enrichment analysis revealed that the metabolites found to be upregulated in IMH individuals were highly enriched for pyrimidine metabolism (*P* < 10-4) and to a lesser extent for other pathways as well (*P* < 0.01) (Figure 3a).

Then, the differences in the expression levels of genes involved in these pathways were analyzed using a cutoff P-value < 0.01 and a pathway impact > 0.05. The analyzed metabolic pathways included pyrimidine metabolism, beta-Alanine metabolism, Pantothenate and CoA biosynthesis and purine metabolism (Figure 3a). This analysis revealed the differential expression of genes involved in the pyrimidine and purine pathways (Figure 8b) but not for beta-Alanine metabolism, nor for the Pantothenate or CoA biosynthesis pathway (*P* < 0.05). In particular, up-regulation of key genes that degrade nucleotide triphosphates (UTP, CTP) to generate uracil and thymine-derived species such as CMPK1, NT5E, UPRT, ENTPD1, and others, was consistent with the metabolite species found in the IMH group. Therefore, integration of metabolomics and gene expression data demonstrate that IMH individuals exhibit signs of nucleotide metabolism dysfunction compared with IML ones.

Considering the importance of oxidative stress in the activation of T cells (through generation of isoketal-modified proteins in dendritic cells), the presence of markers of oxidative stress was analyzed in IMH versus IML subjects (Figure 3b). Using the metabolomics data, it was directly asked whether the levels of circulating cystine (an oxidized product of cysteine) differed between IMH and IML subjects without adjusting for multiple comparisons. Since there is no enzyme that mediates the reaction from cysteine to cystine, this compound is generated from direct reaction with reactive oxygen species (ROS) (Figure 3b) and thus, it is an important marker of oxidative stress. A significant difference in circulating cystine was found in IMH compared with IML subjects (Figure 3c).

**Table 5. List of metabolites up-regulated in IMH compared with IML older subjects. Compounds selected for experiments in primary monocytes are marked with a (*).**

| ***Metabolite name*** | ***Scare(d)*** | ***q-value-(%)*** | ***Metabolite name*** | ***Score(d)*** | ***q-value(%)*** |
|---|---|---|---|---|---|
| stachydrine | 2.449 | 0.000 | orotidine | 1.667 | 13.780 |
| betonicine | 2.429 | 0.000 | xylitol | 1.663 | 13.780 |
| scyllo-inositol * | 2.362 | 0.000 | N3-methyluridine | 1.656 | 13.780 |
| 5,6-dihydrothymine | 2.291 | 0.000 | corticosterone | 1.652 | 13.780 |
| N-acetylthreonine | 2.272 | 0.000 | pseudouridine | 1.644 | 13.780 |
| N4-acetylcytidine * | 2.238 | 0.000 | cholate | 1.642 | 13.780 |
| chiro-inositol | 2.208 | 0.000 | AICA ribonucleotide | 1.636 | 13.780 |
| vanillylmandelate (VMA) * | 2.078 | 0.000 | dimethylglycine | 1.604 | 13.780 |
| N6-methyladernosine | 2.022 | 0.000 | 3-hydroxyhippurate | 1.604 | 13.780 |
| 3-hydroxy-3-methylglutarate | 2.022 | 0.000 | xanthosine | 1.595 | 13.780 |
| 5-adenosylhomocysteine (SAH) | 2004 | 0.000 | N-methylpipecolate | 1.583 | 13.780 |
| acisoga | 1.955 | 0.000 | citrate | 1.582 | 13.780 |
| succinylcarnitine | 1.939 | 0.000 | hexenedioylcarnitine | 1.577 | 13.780 |
| actenine * | 1.431 | 0.000 | N-acetylmethionine | 1.575 | 13.780 |
| N6-carbamoylthreonyladenosine | 1.884 | 0.000 | quinolinate | 1.575 | 13.780 |
| 5,6-dihydrouracil | 1.874 | 0.000 | behenoyl sphingomyelin | 1.567 | 13.780 |
| hypotaurine | 1.873 | 13.780 | gamma-tocopherol | 1.567 | 13.780 |
| 4-acetamidobutanoate | 1.852 | 13.780 | N-acetylalanine | 1.565 | 13.780 |
| 3-ureidopropionate | 1.846 | 13.780 | O-sulfo-L-tyrosine | 1.558 | 13.780 |
| 5-methylthloadenosine (MTA) | 1.844 | 13.780 | 2-aminooctanoate | 1.545 | 13.780 |
| C-glycosyltryptophan | 1.825 | 13.780 | xylonate | 1.541 | 13.780 |
| myo-inositol | 1.811 | 13.780 | fucitol | 1.540 | 13.780 |
| N-acetylserine | 1.778 | 13.780 | 3-methoxytyrosine | 1.500 | 15.966 |
| malonate (propanedioate) | 1.763 | 13.780 | indolebutyrate | 1.495 | 15.966 |
| N6-acetyllysine | 1.760 | 13.780 | 17alpha-hydroxypregnanolone glucuronide | 1.474 | 15.966 |
| pyroglutamine | 1.725 | 13.780 | 3beta,alpha-dihydroxy-5-cholestenoate | 1.473 | 15.966 |
| homovanillate (HVA) | 1.720 | 13.780 | 2-hydroxyphenylacetate | 1.466 | 16.880 |
| N2,N2-dimethylguanes[ne | 1.709 | 13.780 | eicosenoyl sphingomyelin | 1.459 | 16.880 |
| pyridoxine (Vitamin B6) | 1.703 | 13.780 | gulonic acid | 1.453 | 16.880 |
| sucrose | 1.677 | 13.780 | N-methyl proline | 1.445 | 16.880 |
| 4-hydroxyhippurate | 1.676 | 13.780 | 3-(4-hydroxyphenyl)propionate | 1.402 | 16.880 |
| 2-aminoheptanoate | 1.675 | 13.780 | 2-methylmalonyl carnitine | 1.393 | 16.880 |
| ribonate | 1.670 | 13.780 | dimethylmalonic add | 1.375 | 16.880 |
| N-acetylneuraminate | 1.670 | 13.780 | | | |

In addition, we quantified circulating isoketals (8-isoprostane) and found a significant difference in IMH compared with IML subjects (Figure 3d). Together, these findings show that IMH individuals are exposed to higher levels of oxidative stress compared with the IML group. In addition to the defects in nucleotide metabolism, it was hypothesized that metabolic reprogramming of mitochondrial bioenergetics in IMH individuals may lead to constitutive high levels of ROS and subsequent chronic oxidative stress conditions.

### Example 5. Nucleotide metabolites in IMH older adults induce IL1FC production in monocytes and activate primary human platelets

The dysfunction in nucleotide metabolism and in mitochondrial bioenergetics described above may explain the generation of circulating metabolites and chronic oxidative stress, respectively. To study whether the circulating metabolites found in higher levels in the sera from IMH compared to IML subjects up-regulate *NLRC4* gene expression and/or cytokine production, four candidate compounds identified from our analysis were selected. They represent distinct metabolic pathways. These included adenine (purine metabolism), DL-4-hydroxy-3-methoxymandelic acid (vanillylmandelate) (phenylalanine and tyrosine metabolism), scyllo-inositol (inositol metabolism) and N4-acetylcytidine (N4A) (pyrimidine metabolism) (Table 5). Adenosine was included as a positive control for IL1FC production. Primary monocytes from four healthy donors were isolated from fresh blood and incubated with increasing concentrations of the indicated compounds for 6 hours. A significant increase in IL-1α and IL-1β was observed only for the adenosine and adenine treatments, but not with other compounds (Figure 3e). We also tested whether these stimulations lead to changes in the expression of *NLRC4* and *NLRP3* (used as a positive control for adenosine stimulation). As expected, adenosine treatment increased expression of *NLRP3* (Figure 3f). However, no increase in *NLRC4* was observed with this compound. In contrast, treatment with N4A induced both *NLRP3* and *NLRC4* (*P <* 0.01). No effect of adenine, DL-4-hydroxy-3-methoxymandelic acid or scyllo-inositol was observed in expression of these genes (Figure 3f). These results indicate that N4A, an endogenous nucleoside product of degradation tRNA (a marker of oxidative stress), may act as a first priming signal for NLRC4 gene expression, whereas adenosine and adenine may generate a second activation signal for the induction and secretion of IL1FC. More biochemical studies are necessary to address this. The fact that adenine treatment does not up-regulate *NLRP3* or *NLRC4* but induces IL1FC production from primary monocytes, suggests at least a degree of *in vivo* priming with background expression of inflammasome genes in cells from the donors studied here.

In parallel experiments, differentiated THP-1 cells and a human monocytic cell Line were treated to address whether the metabolites N4A and adenine, alone or in combination, could induce inflammasome activation and cytokine secretion *in vitro.* It was found that neither incubation with adenine nor with N4A alone had an effect in the production of IL-1β, IL-18 (another IL1FC) or TNF-α (Figure 4). However, the addition of ATP in presence of N4A potently induced secretion of IL-1β and IL-18, but not TNF-α (Figure 4).

Moreover, co-treating cells with adenine and N4A induced a significant increase in IL-1β and IL-18 levels (Figure 4) which was further augmented after pulsing cells with ATP. This combinatorial effect of N4A and adenine on production of IL-1β and IL-18 was not observed for TNF-α, which indicates that the observed effect is likely dependent on inflammasome activation.

This shows that the presence of both N4-acetylcytidine and adenine may provide both signals necessary for inflammasome activation and secretion of IL1FC from human monocytes.

Platelet activation is a critical step in various inflammatory conditions of both infectious and non-infectious origins and accumulating evidence indicates that hypertensive patients exhibit high levels of activated platelets compared with healthy controls. Putative mechanisms that contribute to platelet activation in hypertension include endothelial dysfunction, neurohumoral (sympathetic and renin-angiotensin systems) overactivity, decreased platelet nitric oxide (NO) biosynthesis, and platelet degranulation secondary to increased shear. An important feature during platelet activation was described in a recent study of dengue infection, where platelets from infected patients or those infected with dengue *in vitro* activated the NLRP3 inflammasome and induced a caspase-1 dependent IL-1β secretion35. Thus, it was sought to determine whether N4A and adenine were able to activate human platelets *in vitro.* To do so, platelets were isolated from the blood of two healthy donors and incubated for 6 hrs at 37°C with various concentrations of adenine or N4A. Thrombin, and ADP were used as controls and platelet activation was monitored by measuring membrane expression of CD61 and CD62P+ cells by flow cytometry. It was observed that adenine robustly induced a dose-dependent increase in the percentage of activated platelets (in contrast to N4A), comparable to the positive control with ADP (Figure 5). This demonstrates that the metabolites identified in IMH subjects are able to activate platelets in addition to human monocytes.

### Example 6. Effect of N4A and adenine on blood pressure in an experimental mouse model

To study whether the selected compounds had a direct effect on blood pressure *in vivo,* mice were injected with N4A plus adenine at the indicated concentrations daily and changes in blood pressure were monitored using a tail cuff method during a total of 34 days. Treatment with N4A and adenine had a mild effect with borderline significant increases in blood pressure (pre-hypertension) as early as 8 days after the first injection (*P* = 0.04 for group comparison) (Figure 6). Based on the previous observation that pre-hypertensive stimuli, such as angiotensin II (Angll), and an oxidative stress-dependent inflammatory response act jointly during sustained hypertension, Angll pumps (at 140 ng/kg/min) were implanted in combination with the compounds (in the treated mice) or with PBS (for the control mice), at day 20. A significant increase was observed in the treated group of mice with an average systolic blood pressure of 140 (±7) vs 112 (±3) mmHg in the control group (*P* = 0.016) (Figure 6). Therefore, these results demonstrate that N4A and adenine can elevate blood pressure in an *in vivo* model.

Overall these data show that the presence of both N4A and adenine is necessary for production of IL1FC from human monocytes, activation of platelets and blood pressure elevation in mice.

### Example 7. Caffeine negatively correlates with expression of inflammasome gene modules 62 and 78

Lowering chronic inflammation in older people may prevent the appearance and delay the clinical symptoms of a number of age-associated diseases. Since adenine and adenosine derivatives were found in high amounts in the IMH group and play a key role in the regulation of IL1FC production, it was asked whether caffeine, a methylxanthine and adenosine antagonist was associated with inflammasome module gene expression. To do so, a questionnaire consisting of a 15-item survey of dietary and pharmaceutical sources of caffeine was administered. For each of the 15 categories in the survey, an approximate caffeine value was derived from 120 of the most commonly consumed caffeinated products in the United States in 2007. A multiple regression analysis was performed using data from all individuals in the year 2008 (N = 89) on the expression of modules 62 and 78 and caffeine intake (in mg/week). For these analyses, it was also adjusted for BMI, a known confounding factor associated with caffeine intake. A significant age-, sex- and BMI-adjusted association was found between caffeine intake and expression of modules 62 (*P* < 0.01) and 78 *(P* = 0.024) (Figure 7a).

We then compared the levels of caffeine and caffeine-derived metabolites in the sera from IMH and IML subjects. To do so, we used the metabolomics data previously generated and directly compared serum levels of caffeine and its metabolites paraxanthine, 1,3,7-trimethyluric acid, theophylline, theobromine and 1-methylxanthine, without adjusting for multiple comparisons. It was found that when considered jointly, the differences for all six compounds combined were statistically significant between the IML and IMH groups (P < 0.01) (Figure 7b).

These results indicate that caffeine intake negatively correlates with expression levels of gene modules 62 and 78 and the circulating levels of this methylxanthine and, when considered jointly, its metabolites are increased in IML subjects compared with IMH ones. Thus, it is possible that moderate coffee consumption may be beneficial to decrease inflammatory processes, by its known effect on the inhibition of adenosine and adenine, which may account in part for the reported correlation with decreased mortality.

### Materials and methods

### Study design, subjects and sample collection

One hundred and fourteen donors (ages 20 to >89) were enrolled in an influenza vaccine study at the Stanford-LPCH Vaccine Program during the years 2008 to 20131-3 (ClinicalTrials.gov registration NCT#01827462). Since baseline samples were obtained from all the individuals prior to vaccination with the influenza vaccine, no randomization or blinding was done for this study. The protocol for this study was approved by the Institutional Review Board of the Research Compliance Office at Stanford University. Informed consent was obtained from all subjects. All individuals were ambulatory and generally healthy as determined by clinical assessment. At the time of initial enrollment volunteers had no acute systemic or serious concurrent illness, no history of immunodeficiency, nor any known or suspected impairment of immunologic function, including clinically observed liver disease, diabetes mellitus treated with insulin, moderate to severe renal disease, blood pressure >150/95 at screening, chronic hepatitis B or C, recent or current use of immunosuppressive medication. In addition, on each annual vaccination day, none of the volunteers had been recipients or donors of blood or blood products within the past 6 months and 6 weeks respectively, and none showed any signs of febrile illness on day of baseline blood draw. Peripheral blood samples were obtained from venipuncture and whole blood was used for gene expression analysis (below). Serum was separated by centrifugation of clotted blood, and stored at -80°C before cytokine and chemokine determination.

### Gene expression analysis

Two different microarray platforms were used to generate expression data from whole blood samples obtained from a total of 114 individuals recruited as part of the Stanford-Ellison cohort1-3; the Human HT12v3 Expression Bead Chip (Illumina, San Diego, CA) for years 2008 and 2009, and the GeneChip PrimeView Human Gene Expression Array (Affymetrix, Santa Clara, CA), for years 2010, 2011 and 2012. For the Illumina platform, biotinylated, amplified antisense complementary RNA (cRNA) targets were prepared from 200 to 250 ng of the total RNA using the Illumina RNA amplification kit (Applied Biosystems/Ambion). Seven hundred and fifty nanograms of labeled cRNA was hybridized overnight to Illumina Human HT-12v3 BeadChip arrays (Illumina), which contained >48,000 probes. The arrays were then washed, blocked, stained and scanned on an Illumina BeadStation 500 following the manufacturer's protocols.

BeadStudio/GenomeStudio software (Illumina) was used to generate signal intensity values from the scans. For normalization, the software was used to subtract background and scale average signal intensity for each sample to the global average signal intensity for all samples. A gene expression analysis software program, GeneSpring GX version 7.3.1 (Agilent Technologies), was used to perform further normalization. For the Affymetrix platform standard Affymetrix 3'IVT Express protocol was used to generate biotinylated cRNA from 50-500ngs of total RNA. DNA polymerase was used for the production of double stranded cDNA. T7 RNA polymerase, in the presence of biotinylated nucleotides, was used for *in vitro* transcription (IVT) of biotinylated cRNA.

The fragmented and labeled targets were hybridized to the PrimeView Human Gene Expression Array cartridge, which measure gene expression of more than 36,000 transcripts and variants per sample by using multiple (11 probes per set for well annotated sequences, 9 probes per set for the remainder) independent measurements for each transcript. The standard Affymetrix hybridization protocol includes 16hr (overnight) hybridization at 45 degree at 60rpm in an Affymetrix GeneChip Hybridization Oven 645. The arrays were then washed and stained in an Affymetrix GeneChip Fluidics Station 450. The arrays were scanned using the Affymetrix GeneChip Scanner 3000 7G and the Affymetrix GeneChip Command Console Software (AGCC) was used for the gene expression data processing and extraction. The raw data for years 2008 through 2012 has been deposited on the Immunology Database and Analysis Portal (ImmPort) under accession numbers SDY314, SDY312, SDY311, SDY112 and SDY315, respectively.

To identify gene modules associated with IL1FC production and inflammasome activity, a list of a total of 89 genes including the Pattern-Recognition Receptor family and their positive and negative regulators encompassing TLRs, NLRs, RIG-I-Like Receptors (RLRs), C-type lectin-like Receptors (CLRs) and their adaptors; inflammatory caspases and their direct regulators; and transcription factors involved in NF-kB and Type-I Interferon (IFN) signaling which are known to regulate inflammasome gene expression and activation was gathered from manually curated data. The presence of these genes was searched across a total of 109 previously defined gene modules. A gene module corresponds to a set of co-expressed genes sharing regulatory programs. Briefly, data were filtered by variance and a total of 6234 highly variant genes were normalized by centering and scaling the expression, so that each gene's expression across all subjects had euclidean norm equal to 1 for purposes of clustering. Data was log transformed to approximate to normal distribution. A hierarchical agglomerative clustering was used with average linkage, euclidean distance and a height cutoff value of 1.5 to derive 109 modules. For each gene module, it was assigned a set of regulatory genes (regulatory program), based on regression analysis of genes in the modules onto expression of known transcription factors using a Akaike Information Criterion (AIC)6. To do so, a linear regression was performed with elastic net penalty of each module's expression onto a set of 188 transcription factors using LARS-EN algorithm. To select the best model among the outputs of LARS-EN, quality of the resulting models by AIC was assessed with sample specific terms weighted by within-module variance. The fit with the best AIC score was selected for each module.

To determine the stability of the age-associations for module 62 and 78, the QuSAGE gene set analysis method was used. It creates a probability distribution representing the mean and standard deviation of a set of genes and enables comparisons of gene sets across different groups. For this analysis, samples from the individuals' first appearance in the study were used to analyze the age associations for module expression.

The presence of extreme phenotypes was examined by using classification based on the magnitude and stability (chronicity) of the expression levels. For each year, the expression of modules 62 and 78 were used to bin subjects into quartiles. Subjects were assigned into inflammasome module high (IMH) or inflammasome module low (IML) groups if they were in the upper (top 25% of subjects) or lower quartile (bottom 25%) in at least in 3/5 years, respectively. Subjects who were not in the upper or lower quartiles in at least 3/5 years were not included in this analysis.

### Determination of cytokines, chemokines and growth factors

**I. Polystyrene bead kits:** Human 50-plex (for year 2008) or 51-plex (for years 2009-2011) kits were purchased from Affymetrix and used according to the manufacturer's recommendations with modifications as described below. Briefly, samples were mixed with antibody-linked polystyrene beads on 96-well filter-bottom plates and incubated at room temperature for 2 h followed by overnight incubation at 4°C. Room temperature incubation steps were performed on an orbital shaker at 500-600 rpm. Plates were vacuum filtered and washed twice with wash buffer, then incubated with biotinylated detection antibody for 2 h at room temperature. Samples were then filtered and washed twice as above and re-suspended in streptavidin-PE. After incubation for 40 minutes at room temperature, two additional vacuum washes were performed, and the samples resuspended in Reading Buffer. Each sample was measured in duplicate. Plates were read using a Luminex 200 instrument with a lower bound of 100 beads per sample per cytokine. Custom assay Control beads by Radix Biosolutions are added to all wells.
**II. Magnetic bead Kits.** Serum specimens were collected from blood samples and frozen in aliquots at -80°C. Human 63-plex (for year 2013) kits were purchased from eBiosciences/Affymetrix, of which 62 analytes passed QC; and used according to the manufacturer's recommendations with modifications as described below. Briefly: beads were added to a 96 well plate and washed in a Biotek ELx405 washer. Samples were added to the plate containing the mixed antibody-linked beads and incubated at room temperature for 1 hour followed by overnight incubation at 4°C with shaking. Cold and Room temperature incubation steps were performed on an orbital shaker at 500-600 rpm. Following the overnight incubation plates were washed in a Biotek ELx405 washer and then biotinylated detection antibody added for 75 minutes at room temperature with shaking. Plate was washed as above and streptavidin-PE was added. After incubation for 30 minutes at room temperature wash was performed as above and reading buffer was added to the wells. Each sample was measured in duplicate.

Plates were read using a Luminex 200 instrument with a lower bound of 50 beads per sample per cytokine. Custom assay Control beads by Radix Biosolutions are added to all wells. Mean fluorescence intensities (MFIs) were recorded and used for further analysis. To identify differences between IH and IL individuals in an unbiased fashion, data were analyzed from year 2013 since this was the year with the largest number of measured analytes (N = 62). A multiple regression analysis was conducted on each analyte' MFI against IH/IL status, age and sex and obtained significance for each regression coefficient via permutation tests over 500 resamplings. To study whether the differences in IL-1α and IL-1β observed in IH subjects compared to IL ones were longitudinally stable, the levels of IL-1α and IL-1β from data generated in the years 2008 through 2011 were compared between IH and IL subjects, using regression model with IL-1β or IL-1α MFI against IH/IL status, age and sex without multiple hypothesis correction.

Combined data showed homoscedasticity based on Bartlett's test. Cytokine data from 2012 was not included in this analysis because data from only 14 extreme phenotype individuals was available. P-values for years 2008 through 2011 were combined using a modified generalized Fisher method for combining P-values from dependent tests.

### Cardiovascular phenotyping

A subgroup of patients (*N =* 17) from the Stanford-Ellison cohort underwent comprehensive cardiovascular assessment at Stanford Cardiovascular Institute Biomarker and Phenotypic Core Laboratory. Vascular studies included the measurement of both carotid intima-media thickness (cIMT) and central aortic pulse wave velocity (PWV). A 9.0 MHz Philips linear array probe was used for carotid and femoral measurements. The cIMT was the average of the anterior, lateral, and posterior measurements and averaged for both the right and left carotid artery. Aortic PWV was calculated as the path length travelled and divided by transit time of the aortic pulse wave and reflects arterial stiffness. Path length (D) was measured as the distance from the sternal notch to the femoral artery minus the echocardiographic distance from the sternal notch to proximal descending aorta.

### Metabolomics data generation and analysis

Metabolomic data were conducted at Metabolon as described previously using nontargeted metabolomic profiling. Briefly, serum samples from IMH (*N* = 11) and IML (*N* = 9) were subjected to methanol extraction then split into aliquots for analysis by ultrahigh performance liquid chromatography/mass spectrometry (UHPLC/MS) in the positive, negative or polar ion mode and by gas chromatography/mass spectrometry (GC/MS). Metabolites were identified by automated comparison of ion features to a reference library of chemical standards followed by visual inspection for quality control. For statistical analyses and data display, any missing values were assumed to be below the limits of detection; these values were imputed with the compound minimum (minimum value imputation). To determine statistical significance, Significance Analysis of Microarrays (SAM)12 was conducted on the residuals from a multiple regression model which included age and sex as covariates. A *Q*-value < 0.05 was used as an indication of high confidence in a result. A total of 67 differentially regulated metabolites were observed in IML versus IMH individuals. Pathway analysis was conducted using MetPA13 which combines several advanced pathway enrichment analysis along with the analysis of pathway topological characteristics across over 874 metabolic pathways. For over representation and pathway topology analyses, hypergeometric test and relative-betweeness centrality were used, respectively.

### Differential expression of purine and pyrimidine metabolism genes

A total of 104 pyrimidine metabolism genes (PYR) and 54 genes participating in purine metabolism (PUR) were obtained from KEGG14. Regression analysis was conducted on each gene's expression using microarray data from year 2008 against IML/IMH status, while adjusting for age and sex. Significance for each regression coefficient was obtained via permutation tests. Genes differentially expressed were subjected to enrichment analysis by hypergeometric test. A P-value < 0.05 was used as an indication of high confidence in a result.

### Compound treatment, cytokine secretion and qPCR assays

Adenosine, adenine, DL-4-hydroxy-3-methoxymandelic acid, scyllo-inositol were all purchased form Sigma (Sigma-Aldrich, St. Louis, MO) and N4-acetylcytidine was purchased from Santa Cruz Biotechnology (Dallas, TX). Compounds were tested at the indicated concentrations on isolated monocytes from a healthy donor. Whole blood was obtained from venipuncture (30 ml) and monocytes were enriched using the RosetteSepTM Human Monocytes Enrichment Cocktail (cat # 15068, Stemcell Technologies, Vancouver, BC, Canada) according to the manufacturer's recommendations. Cells were plated on 96-well plates at a density of 3 x 10-5 cells in 200 uL LGM-3 serum-free media (Lonza) and incubated for 6 hours at 37°C. Supernatants were collected, frozen immediately and stored at -80°C. Samples were then transferred to the Human Immune Monitoring Core at Stanford for quantification of cytokines, chemokines and growth factors using the 63-plex Luminex system, as described above. To assess significance for the dose-response experiments we used Short Time-series Expression Miner (STEM) which uses clustering methods for time-series or dose response experiments and allows for the identification of significant dose-dependent profiles.

RNA was extracted from cell pellets using the RNeasy Micro Kit (Qiagen) following the manufacturer's recommendations. cDNA was prepared using the SuperScript^{®} VILOTM cDNA Synthesis Kit (Life Technologies). *NLRC4* and *NLRP3* expression was measured by quantitative PCR using pre-design TaqMan^{®} Gene Expression Assays (Life Technologies) and plates were run on a StepOneTM Real Time PCR System (Applied Biosciences). Expression of GAPDH was used to standardize the samples, and the results are expressed as a ratio relative to control.

### Hypertension studies in mice

Adult male mice (12-18 week old) were divided into two groups: PBS or compound treated-mice (N4-Acetylcytidine + Adenine dissolved in PBS). Compound-treated mice were injected with N4-Acetylcytidine and Adenine (stock solution 20 mM each, 100 ul/25 g body weight, retro-orbital injection, once daily) or PBS. After 3 weeks of treatment, while they continued receiving daily injections of PBS or N4A+Adenine, mice from both groups were administered an infusion of human angiotensin II (Angll, #A9525, Sigma-Aldrich, St. Louis, MO) for another 2 weeks. Angll (140 ng/kg per min) was dissolved in 100 ul 20 mM (N4A + Adenine) or in 100 ul PBS and loaded into a small osmotic pump (Durect Corporation, Cupertino, CA, USA). The osmotic pump was then implanted subcutaneously on the dorsal side around the neck of mice under anesthesia (2% oxygen, 2.5% isoflurane). Systolic blood pressure was measured every other day in conscious mice using tail-cuff plethysmography (Vistech System BP-2000, Apex, NC, USA).

### Experiments with human THP-1 cells and primary blood platelets

THP-1 monocytic cell lines were cultured in 6-well plates in RPMI media (supplemented with 10% Fetal Bovine Serum) and differentiated overnight with TPA (10 ng/ml). The day after, adherent cells were washed with fresh media and treated with agonists LPS (1 ng/ml, 4 hrs) and ATP (5 mM, 30 min) or compounds Adenine/N4-Acetylcytidine at various concentrations.

Human primary platelets were prepared from whole blood using a venepuncture in EDTA tube after a 20 min centrifugation at 1000 rpm without acceleration and break. Then the supernatant was harvested and 1µM PGE1 was added. After gentle centrifugation (10 min at 2000 rpm without break), supernatant was removed and Tyrode buffer was added. Platelets were then stimulated with thrombin (at 0.5 U/ml), ADP, or with the indicated concentrations of N4A or adenine, and activation was monitored by flow cytometry using immunostaining of membrane markers with anti-CD61 (marker of platelet population), and anti-CD62-P (marker of activation involved in aggregation).

## Claims

1. Use of at least one nucleotide-derived metabolite selected from the group consisting of N4-acetylcytidine and adenine in the *in vitro*/*ex vivo* diagnosis of a disease in a sample obtained from a subject, said disease being an inflammation-related disease.

2. Use according to claim 1, wherein said disease is an inflammasome-related disease.

3. Use according to any one of claims 1 or 2, wherein said disease is a chronic inflammation, preferably a low-grade chronic inflammation, or a cardiovascular disease, preferably hypertension.

4. Use according to any one of claims 1 or 2, wherein said disease is a cardiovascular disease induced by a chronic inflammation.

5. Use according to any one of claims 1 to 4, wherein said subject is a human at least 60 years old.

6. Use according to any one of claims 1 to 5, wherein said sample is blood, serum, plasma, urine, preferably serum.

7. Use according to any one of claims 1 to 6, wherein the concentration of said at least one nucleotide-derived metabolite is determined using an assay selected from the group consisting of immunoassays, aptamer-based assays and mass spectrometry-based assays.

8. Method for *in vitro*/*ex vivo* diagnosing a disease in a subject, said disease being an inflammation-related disease, said method comprising the step of determining the concentration of at least one nucleotide-derived metabolite selected from the group consisting of N4-acetylcytidine and adenine, in a sample obtained from said subject.

9. Method according to claim 8, wherein said disease is an inflammasome-related disease.

10. Method according to any one of claims 8 or 9, wherein said disease is a chronic inflammation, preferably a low-grade chronic inflammation, or a cardiovascular disease, preferably hypertension.

11. Method according to any one of claims 8 or 9, wherein said disease is a cardiovascular disease induced by a chronic inflammation.

12. Method according to any one of claims 8 to 11, wherein said subject is a human at least 60 years old.

13. A screening method for determining whether a compound would be effective in the treatment of a disease, said disease being an inflammation-related disease, comprising:
- a step of incubating said compound *in vitro* with cells that produce at least one nucleotide-derived metabolite selected from the group consisting of N4-acetylcytidine and adenine,
- a step of determining the extent of decrease caused by said compound on the production of said at least one nucleotide-derived metabolite.

14. Method of monitoring treatment efficacy in a subject undergoing treatment for a disease, said disease being an inflammation-related disease, said method comprising:
- determining the concentration of at least one nucleotide-derived metabolite selected from the group consisting of adenine and N4-acetylcytidine, in samples obtained from said subject over time, and
- determining the evolution of said concentration of at least one nucleotide-derived metabolite, whereby:
said treatment is effective if said concentration of at least one nucleotide-derived metabolite decreases overtime, and
said treatment is ineffective if said concentration of at least one nucleotide-derived metabolite is stable or increases over time.

15. Kit for diagnosing a disease in a sample, said disease being an inflammation-related disease, comprising:
one or more reagent allowing the detection of at least one nucleotide-derived metabolite selected from the group consisting of N4-acetylcytidine and adenine.
